(19) Europäisches Patentamt
European Patent Office
Office européen des brevets

(11) Veröffentlichungsnummer: **0 279 783**
**A1**

# (12) EUROPÄISCHE PATENTANMELDUNG

(21) Anmeldenummer: **88810088.0**

(22) Anmeldetag: **12.02.88**

(51) Int. Cl.4: **C 07 D 493/22**
A 01 N 43/90, A 61 K 31/35
//(C07D493/22,313:00,311:00,
311:00,307:00)

(30) Priorität: **18.02.87 CH 600/87**

(43) Veröffentlichungstag der Anmeldung:
**24.08.88 Patentblatt 88/34**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Frei, Bruno, Dr.**
**Bündtenstrasse 16**
**CH-4410 Liestal (CH)**

**O'Sullivan, Anthony Cornelius, Dr.**
**Habsburgerstrasse 38**
**CH-4055 Basel (CH)**

## (54) Insektizide und Parasitizide.

(57) Die vorliegende Erfindung betrifft neue 5-Oximino-13β-carbonylthiomilbemycin-Derivate der Formel I, ihre Herstellung und ihre Verwendung zur Bekämpfung von Schädlingen, ferner Schädlingsbekämpfungsmittel, die mindestens eien dieser Verbindungen als Wirkstoff enthalten.

Die neuen Verbindungen haben die allgemeine Formel I

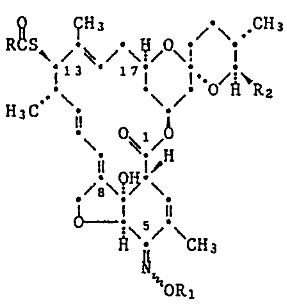

(I)

in welcher
$R_1$ Wasserstoff, eine Alkyl-, Cycloalkyl- oder Acylgruppe bedeutet,
$R_2$ für Methyl, Ethyl, Isopropyl oder sek.Butyl oder für die Gruppe 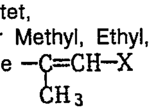 steht, worin X Methyl, Ethyl oder Isopropyl bedeutet und R Wasserstoff, eine unsubstituierte oder substituierte gerad- oder verzweigtkettige $C_1$-$C_{18}$-Alkyl-Gruppe, eine unsubstituierte oder substituierte cycloaliphatische Gruppe mit 3 bis 10 Kohlenstoffatomen, eine unsubstituierte oder substituierte $C_2$-$C_6$-Alkenyl-Gruppe, eine unsubstituierte oder substituierte $C_2$-$C_6$-Alkinyl-Gruppe, eine unsubstituierte oder substituierte Benzylgruppe bedeutet.

EP 0 279 783 A1

**Beschreibung**

Insektizide und Parasitizide

Die vorliegende Erfindung betrifft neue 5-Oximino-13β-carbonylthiomilbemycin-Derivate der Formel I, ihre Herstellung und ihre Verwendung zur Bekämpfung von Schädlingen, ferner Schädlingsbekämpfungsmittel, die mindestens eine dieser Verbindungen als Wirkstoff enthalten.

Die neuen Verbindungen haben die allgemeine Formel I

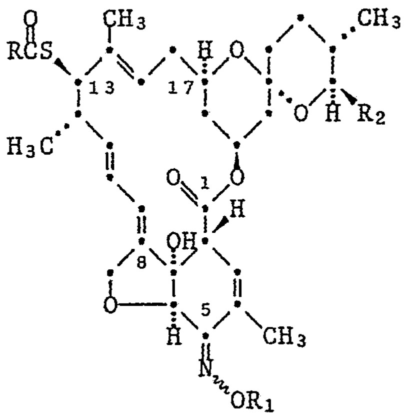

(I)

in welcher,
$R_1$ Wasserstoff, eine Alkyl-, Cycloalkyl- oder Acylgruppe bedeutet, $R_2$ für Methyl, Ethyl, Isopropyl oder sek.Butyl oder für die Gruppe $-\underset{CH_3}{C}=CH-X$ steht, worin X Methyl, Ethyl oder Isopropyl bedeutet und

R Wasserstoff, eine unsubstituierte oder substituierte gerad- oder verzweigtkettige $C_1$-$C_{18}$-Alkyl-Gruppe, eine unsubstituierte oder substituierte cycloaliphatische Gruppe mit 3 bis 10 Kohlenstoffatomen, eine unsubstituierte oder substituierte $C_2$-$C_6$-Alkenyl-Gruppe, eine unsubstituierte oder substituierte $C_2$-$C_6$-Alkinyl-Gruppe, eine unsubstituierte oder substituierte Phenyl-Gruppe oder eine unsubstituierte oder substituierte Benzylgruppe bedeutet.

Ganz besonders bevorzugt im Rahmen dieser Erfindung sind die neuen Verbindungen der allgemeinen Formel Ia

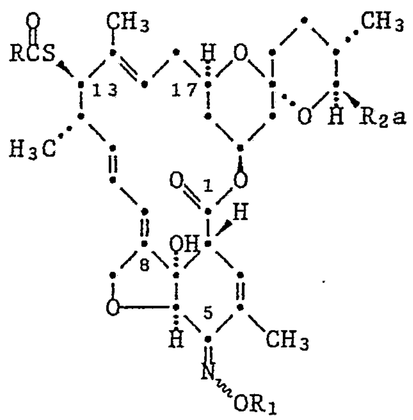

(Ia)

in welcher,
$R_1$ Wasserstoff, eine Alkyl-, Cycloalkyl- oder Acylgruppe,
$R_{2}a$ Methyl, Ethyl, Isopropyl oder sek.Butyl und
R Wasserstoff, eine unsubstituierte oder substituierte gerad- oder verzweigtkettige $C_1$-$C_{18}$-Alkyl-Gruppe, eine unsubstituierte oder substituierte cycloaliphatische Gruppe mit 3 bis 10 Kohlenstoffatomen, eine unsubstituierte oder substituierte $C_2$-$C_6$-Alkenyl-Gruppe, eine unsubstituierte oder substituierte $C_2$-$C_6$-Alkinyl-Gruppe, eine unsubstituierte oder substituierte Phenyl-Gruppe oder eine unsubstituierte oder substituierte Benzylgruppe bedeutet.

Unter den vorgenannten Bedeutungen sind als bevorzugt anzusehen: unsubstituiertes oder halogeniertes $C_1$-$C_{18}$-Alkyl, unsubstituiertes oder durch Methyl ein- oder mehrfach substituiertes $C_3$-$C_6$-Cycloalkyl, Adamantyl, unsubstituiertes oder halogeniertes $C_2$-$C_6$-Alkenyl, $C_2$-$C_6$-Alkinyl, unsubstituiertes oder substitu-

iertes Phenyl-Gruppen oder unsubstituierte oder substituierte Benzylgruppen.

Als Substituenten der Alkyl-, Cycloalkyl-, Alkenyl- und Alkinyl-Gruppen kommen beispielsweise 1 bis 7 Halogenatome oder 1 bis 6 $C_1$-$C_6$-Alkylthio- und $C_1$-$C_6$-Alkoxygruppen und als Substituenten der Phenyl- und Benzyl-Gruppen 1 bis 3 Substituenten ausgewählt aus der Gruppe bestehend aus Halogenatomen, $C_1$-$C_6$-Alkyl, $C_1$-$C_6$-Alkoxy, $C_1$-$C_4$-Alkylthio, Haloalkyl und Nitro in Betracht. Der Phenylring kann auch durch eine Difluormethylendioxigruppe substituiert sein, wobei die beiden Sauerstoffatome an direkt benachbarten Kohlenstoffatomen des Phenylrings sitzen. Als Substituent der mit dem Carboxylsäurerest unmittelbar verbundenen Alkylgruppe kommt ausserdem auch eine unsubstituierte oder substituierte Phenyl- oder Phenoxygruppe in Frage, wie beispielsweise eine halogenierte, insbesondere eine durch 1 bis 3 Halogenatome substituiertes Phenyl- oder Phenoxygruppe. Bei den Cycloalkylgruppen können als Substituenten auch $C_1$-$C_4$-Alkylgruppen vorliegen, bei Cyclopropyl auch eine 2,2-Dichlorvinylgruppe.

Unter dem Begriff Alkyl als Substituent oder Teil eines Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl oder Decyl, sowie die Isomeren, wie beispielsweise Isopropyl, Isobutyl, tert.-Butyl oder Isopentyl. Haloalkyl steht für einen einfach bis perhalogenierten Alkylsubstituenten, wie beispielsweise $CHCl_2$, $CHF_2$, $CH_2Cl$, $CCl_3$, $CF_3$, $CH_2F$, $CH_2CH_2Cl$, $CHBr_2$. Unter Halogen soll hier und im folgenden Fluor, Chlor, Brom oder Jod, vorzugsweise Fluor, Chlor oder Brom, verstanden werden. Als Cycloalkyl-Gruppen kommen mono- bis tetracyclischen Gruppen in Betracht, wie beispielsweise Cyclopropyl, Cyclobutyl, Cyclopentyl, Cyclohexyl, Decalin, Hydrindan, Bicycloheptan, Bicyclooctan, Norbornan, Bornan oder Adamantyl. Diese cycloaliphatischen Gruppen sind vorzugsweise unsubstituiert oder durch Methyl ein- oder mehrfach substituiert. Alkenyl steht für einen durch mindestens eine C=C-Doppelbindung charakterisierten aliphatischen, acyclischen Kohlenwasserstoffrest, wie beispielsweise für Vinyl, Propenyl-(1), Allyl, Butenyl-(1), Butenyl-(2) oder Butenyl-(3). Haloalkenyl bedeutet dementsprechend einen derartigen Alkenylrest, der ein- oder mehrfach halogniert ist. Alkinyl steht für eine gerade oder verzweigte Kohlenstoffkette, die durch mindestens eine C≡C Dreifachbindung charakterisiert ist. Typische Vertreter sind beispielsweise Ethinyl, Propinyl-(1), Propargyl oder Butinyl-(1). Alkoxyalkyl steht für eine unverzweigte oder verzweigte, durch ein Sauerstoffatom unterbrochene Alkylgruppe, beispielsweise für $CH_2OCH_3$, $CH_2CH_2OCH_3$, $CH_2CH(CH_3)OCH_3$, $CH_2OC_2H_5$, $C(CH_3)_2OCH_3$, $CH_2OC_3H_7$-i oder $CH_2CH_2CH_2OCH_3$.

Als substituiertes Phenyl kommen beispielsweise 2,6-Dimethylphenyl, 2,4-Dichlorphenyl, 2,3,6-Trichlorphenyl, p-Bromphenyl, 2,4-Xylyl, 3-Nitrophenyl, 4-Chlor-2-methylphenyl, 4-Methyl-2-methoxyphenyl, 2,4,6-Trimethylphenyl oder p-Methylthiophenyl in Betracht.

Als Beispiele für R, die keine Limitierung darstellen, sind zu nennen: Wasserstoff, Methyl, Ethyl, Propyl, Isopropyl, n-Butyl, sek.Butyl, tert.Butyl, Neopentyl, Chlormethyl, Trifluormethyl, Trichlormethyl, Trichlorethyl, Trichlor-tert.Butyl, 1,2,2,2-Tetrachlorethyl, 1,3,3,3-Tetrachlorpropyl, 3-Chlorpropyl, Ethenyl, Propenyl, Methoxymethyl, Isopropoxymethyl, 1-Methyl-1-methoxyethyl, 2,2-Dimethylvinyl, 1,2,2-Trichlorvinyl, 1,3,3,3-Tetrachlorpropyl, 1,1-Dichlor-2,2,2-trifluorethyl, 1,3-Pentadienyl, Ethinyl, 1-Propinyl, 1-Butinyl, Cyclopropyl, 2,2-Dimethylcyclopropyl, 1-Methylcyclopropyl, 2,2-Dimethyl-3-(2,2-dichlorvinyl)-cyclopropyl, Cyclobutyl, Cyclopentyl, Cyclohexyl, Adamantyl, Phenyl, Benzyl, p-Tolyl, p-Chlorphenyl, 2,6-Dichlorphenyl oder 2,4-Dinitrophenyl oder 4-Fluorphenoxymethyl, 2-Trifluormethylphenyl, 2,6-Dimethylphenyl, 2-Phenylethyl, 1-Methyl-1-phenylethyl, 1-Methoxi-1-methylethyl und 1-Methyl-1-thiomethylethyl.

Verbindungen der Formel I, worin $R_1$ Wasserstoff bedeutet, sind bevorzugt. Geeignete Acylgruppen sind beispielsweise die Reste $R_3$-C(O)-, wobei $R_3$ für $C_1$-$C_{10}$-Alkyl, $C_1$-$C_{10}$-Haloalkyl oder einen unsubstituierten oder durch Substituenten der Gruppe bestehend aus Halogen, $C_1$-$C_3$-Alkyl, $C_1$-$C_3$-Haloalkyl, $C_1$-$C_3$-Alkoxy, $C_1$-$C_3$-Halo alkoxy, Cyano und Nitro substituierten Rest aus der Gruppe Phenyl und Benzyl steht und vorzugsweise $C_1$-$C_6$-Alkyl, $C_1$-$C_6$-Haloalkyl oder unsubstituiertes oder durch Halogen, $C_1$-$C_3$-Alkyl, $CF_3$ oder Nitro substituiertes Phenyl bedeutet.

Verbindungen, worin $R_2$ sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13α-Position) lassen sich jedoch gemäss US-PS 4,173,571 in Milbemycin-Homologe überführen.

In natürlich vorkommenden Milbemycin ($R_2 = CH_3$, $C_2H_5$ oder iso$C_3H_7$) ist die 13-Position anstelle der Thioestergruppe der erfindungsgemässen Verbindungen der Formel I ausschliesslich mit Wasserstoff besetzt, wie die nachstehende Formel XXX zeigt:

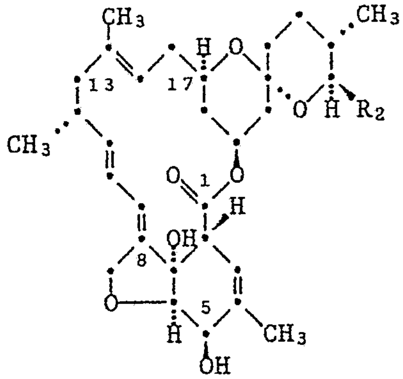

(XXX)

| | | |
|---|---|---|
| $R_2$ | $= CH_3$ | Milbemycin $A_3$ |
| $R_2$ | $= C_2H_5$ | Milbemycin $A_4$ |
| $R_2$ | $= isoC_3H_7$ | Milbemycin D |
| $R_2$ | $= sec.C_4H_9$ | 13-Deoxi-22,23-dihydro-C-076-Bla-aglycon. |

Bei Avermectinen dagegen steht in der 13-Positionen ein α-L-Oleandrosyl-α-L-oleandrose-Rest, der über Sauerstoff in α-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen Substituenten $R_2$ = sek.$C_4H_9$ von den Milbemycinen. Durch Hydrolyse des Zucker-Restes der Avermectinen gelangt man leicht zu den entsprechenden Avermectin-Aglykonen, die eine in Nachbarstellung zu einer C=C-Doppelbindung befindliche 13α-Hydroxy-Gruppe besitzen. Die Avermectin-Aglykone sind, wie vorstehend angegeben, in die Milbemcyin-Homologen unwandelbar. Bei den Milbemycin-Derivaten der vorliegenden Anmeldung liegt die $\Delta^{22,23}$-Doppelbindung stets in hydrierter Form vor und der Substituent in 13-Position ist immer β-ständig.

Verbindungen der Formel I, worin $R_2$ für die Gruppe 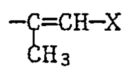 steht und X Methyl, Ethyl oder Isopropyl bedeutet, repräsentieren solche 23-Deoxy-Abkömmlinge der natürlich vorkommenden Antibiotika S541, die nunmehr in 13-Position eine β-Carbonylthio- und in 5-Position eine Oximino-Gruppierung enthalten.

Die Konstitution von natürlichen Antibiotika S541 ist aus der DE 35 32 794 bekannt und sieht wie folgt aus:

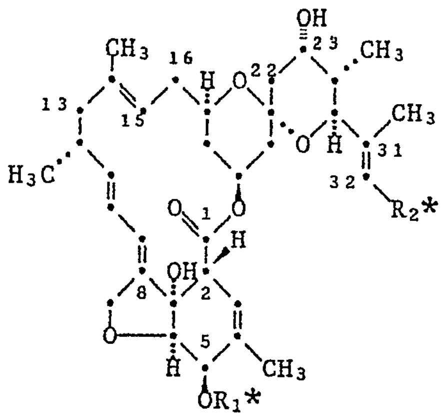

(Antibiotika S541)

| | | |
|---|---|---|
| Faktor A | $R_2^*=isoC_3H_7$ | $R_1^*=H$ |
| Faktor B | $R_2^*=CH_3$ | $R_1^*=CH_3$ |
| Faktor C | $R_2^*=CH_3$ | $R_1^*=H$ |
| Faktor D | $R_2^*=C_2H_5$ | $R_1^*=H$ |
| Faktor E | $R_2^*=C_2H_5$ | $R_1^*=CH_3$ |
| Faktor F | $R_2^*=isoC_3H_7$ | $R_1^*=CH_3$ |

Folgende Untergruppen von Verbindung der Formel I sind auf Grund ihrer ausgeprägten parasitiziden und insektizidin Wirkung besonders bevorzugt:

Gruppe Ia:

Verbindungen der Formel I, worin $R_1$ Wasserstoff darstellt, $R_2$ für Methyl, Ethyl, Isopropyl oder sek.Butyl oder die Gruppe $-\underset{CH_3}{C}=CH-X$ steht, worin X Methyl, Ethyl oder Isopropyl bedeutet

und R folgende Bedeutungen hat:
- Wasserstoff, unsubstituiertes oder durch 1 bis 4 Halogenatome, $C_1$-$C_4$-Alkylthio oder $C_1$-$C_4$-Alkoxy substituiertes $C_1$-$C_6$-Alkyl, $C_2$-$C_4$-Alkenyl, $C_2$-$C_4$-Alkinyl oder $C_3$-$C_6$-Cycloalkyl;
- unsubstituiertes oder ein bis dreifach durch Halogen, $C_1$-$C_4$-Alkyl, $C_1$-$C_4$-Alkoxy, $C_1$-$C_4$-Alkylthio oder Nitro substituiertes Phenyl oder Benzyl;

Gruppe Ib:

Verbindungen der Formel I, worin $R_1$ Wasserstoff darstellt, $R_2$ für Methyl, Ethyl, Isopropyl oder sek.Butyl oder die Gruppe $-\underset{CH_3}{C}=CH-X$ steht, worin X Methyl, Ethyl oder Isopropyl bedeutet und R folgende Bedeutungen hat:
- Wasserstoff, unsubstituiertes oder durch 1 bis 4 Chlor- oder Fluoratome, Methylthio oder Methoxy substituiertes $C_1$-$C_4$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl oder Benzyl;

Gruppe Ic:

Verbindungen der Formel I, worin $R_1$ Wasserstoff darstellt, $R_2$ für Methyl, Ethyl, Isopropyl oder sek.Butyl steht und R folgende Bedeutungen hat:
- Wasserstoff, unsubstituiertes oder durch 1 bis 4 Halogenatome, $C_1$-$C_4$-Alkylthio oder $C_1$-$C_4$-Alkoxy substituiertes $C_1$-$C_6$-Alkyl, $C_2$-$C_4$-Alkenyl, $C_2$-$C_4$-Alkinyl oder $C_3$-$C_6$-Cycloalkyl;
- unsubstituiertes oder ein bis dreifach durch Halogen, $C_1$-$C_4$-Alkyl, $C_1$-$C_4$-Alkoxy, $C_1$-$C_4$-Alkylthio oder Nitro substituiertes Phenyl oder Benzyl;

Gruppe Id:

Verbindungen der Formel I, worin $R_1$ Wasserstoff darstellt, $R_2$ für Methyl, Ethyl, Isopropyl oder sek.Butyl steht und R folgende Bedeutungen hat:
- Wasserstoff, unsubstituiertes oder durch 1 bis 4 Chlor- oder Fluoratome, Methylthio oder Methoxy

substituiertes $C_1$-$C_4$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl oder Benzyl;

Gruppe Ie:

Verbindungen der Formel I, worin $R_1$ Wasserstoff darstellt, $R_2$ für Methyl oder Ethyl steht und R folgende Bedeutungen hat:
- Wasserstoff, unsubstituiertes oder durch 1 bis 4 Chlor- oder Fluoratome, Methylthio oder Methoxy substituiertes $C_1$-$C_4$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl oder Benzyl;

Gruppe If:

Verbindung der Formel I, worin $R_1$ Wasserstoff darstellt, $R_2$ für Isopropyl oder sek.Butyl steht und R folgende Bedeutungen hat:
- Wasserstoff, unsubstituiertes oder durch 1 bis 4 Chlor- oder Fluoratome, Methylthio oder Methoxy substituiertes $C_1$-$C_4$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl oder Benzyl;

Gruppe Ig:

Verbindungen der Formel I, worin $R_1$ Wasserstoff darstellt, $R_2$ für Methyl, Ethyl, Isopropyl oder sek.Butyl oder die Gruppe $-\underset{CH_3}{\underset{|}{C}}=CH-X$ steht, worin X Methyl, Ethyl oder Isopropyl bedeutet und R folgende Bedeutungen hat:
- Wasserstoff, unsubstituiertes oder durch 1 bis 3 Chloratome oder Fluoratome, Methylthio oder Methoxy substituiertes $C_1$-$C_4$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl oder Benzyl;

Gruppe Ih:

Verbindungen der Formel I, worin $R_1$ Wasserstoff darstellt, $R_2$ für Methyl, Ethyl, Isopropyl oder sek.Butyl steht und R folgende Bedeutungen hat:
- Wasserstoff, unsubstituiertes oder durch 1 bis 3 Chloratome oder Fluoratome, Methylthio oder Methoxy substituiertes $C_1$-$C_4$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl oder Benzyl;

Gruppe Ii:

Verbindungen der Formel I, worin $R_1$ Wasserstoff darstellt, $R_2$ für Methyl oder Ethyl steht und R folgende Bedeutungen hat:
- Wasserstoff, unsubstituiertes oder durch 1 bis 3 Chloratome oder Fluoratome, Methylthio oder Methoxy substituiertes $C_1$-$C_4$-Alkyl, $C_2$-$C_3$-Alkenyl, $C_2$-$C_3$-Alkinyl oder $C_3$-$C_6$-Cycloalkyl.

Gruppe Ik:

Verbindungen der Formel I, worin $R_1$ Wasserstoff darstellt, $R_2$ für Isopropyl oder sek.Butyl steht und R folgende Bedeutungen hat:
- Wasserstoff, unsubstituiertes oder durch 1 bis 3 Chloratome oder Fluoratome oder Methoxy substituiertes $C_1$-$C_4$-Alkyl, $C_2$-$C_3$-Alkenyl, $C_2$-$C_3$-Alkinyl oder $C_3$-$C_6$-Cycloalkyl.

Gruppe Il:

Verbindungen der Formel I, worin $R_1$ Wasserstoff darstellt, $R_2$ für Ethyl oder Methyl steht und R folgende Bedeutungen hat: Wasserstoff, $C_1$-$C_8$-Alkyl, welches unsubstituierte oder durch $C_1$-$C_4$-Alkoxy, $C_1$-$C_4$-Alkylthio, Phenyl oder ein- bis dreifach halogeniertes Phenyl oder Phenoxy monosubstituiert oder durch 1 bis 5 Halogenatome substituierte ist, eine unsubstituierte oder durch Substituenten aus der Gruppe bestehend aus $C_1$-$C_4$-Alkyl und halogeniertem $C_2$-$C_4$-Alkenyl ein- oder mehrfach substituierte, mono- bis tetracyclische, aliphatische Gruppe mit insgesamt 3 bis 10 Kohlenstoffatomen im Ring oder Ringsystem, ein-bis dreifach halogeniertes $C_2$-$C_4$-Alkenyl, $C_3$-$C_4$-Alkinyl oder ein-bis dreifach durch Substituenten aus der Gruppe bestehend aus Halogen, $C_1$-$C_4$-Alkyl und Nitro substituiertes Phenyl oder Benzyl.

Gruppe Im:

Verbindungen der Formel I, worin $R_1$ Wasserstoff darstellt, $R_2$ für Ethyl oder Methyl steht und R folgende Bedeutungen hat: Wasserstoff, $C_1$-$C_8$-Alkyl, welches unsubstituierte oder durch Substituenten der Gruppe bestehend aus Chlor und Fluor ein- bis dreifach substituiert ist, Fluorphenoxymethyl, unsubstituiertes oder durch eine Methylgruppe substituiertes $C_3$-$C_4$-Cycloalkyl, Adamantyl, Trichlorvinyl, Phenyl oder Monochlorphenyl.

Besonders bevorzugte 5-Oximino-Derivate der Formel I worin $R_1$ Wasserstoff bedeutet sind beispielsweise:
13β-Formylthio-5-oximino-milbemycin D
13β-Acetylthio-5-oximino-milbemycin D
13β-Pivaloylthio-5-oximino-milbemycin D
13β-Formylthio-5-oximino-milbemycin $A_3$
13β-Acetylthio-5-oximino-milbemcyin $A_3$
13β-Pivaloylthio-5-oximino-milbemycin $A_3$
13β-Formlythio-5-oximino-milbemycin $A_4$
13β-Acetylthio-5-oximino-milbemycin $A_4$
13β-Pivaloylthio-5-oximino-milbemycin $A_4$.
13β-(2′-Methoxy-2′-methylpropionylthio)-5-oximino-milbemycin D
13β-(2′-Methoxy-2′-methylpropionylthio)-5-oximino-milbemycin $A_4$
13β-Trichloracetylthio-5-oximino-milbemycin $A_4$
13β-(4′-Chlor-butanoylthio)-5-oximino-milbemycin $A_4$
13β-Trichloracryloylthio-5-oximino-milbemycin $A_4$
13β-Cyclopropancarbonylthio-5-oximino-milbemycin $A_4$
13β-Cyclobutancarbonylthio-5-oximino-milbemycin $A_4$
13β-Heptanoylthio-5-oximino-milbemycin $A_4$
13β-(3′-Chlor-2′,2′-dimethyl-propionyltho)-5-oximino-milbemycin $A_4$
13β-(3′-Chlor-2′,2′-dimethyl-propionylthio)-5-oximino-milbemycin $A_3$
13β-(1′-Methyl-cyclopropancarbonylthio)-5-oximino-milbemycin $A_4$
13β-(1′-Methyl-cyclopropancarbonylthio)-5-oximino-milbemycin $A_3$
13β-(1-Adamantancarbonylthio)-5-oximino-milbemycin $A_4$
13β-(p-Fluorphenoxyacetylthio)-5-oximino-milbemycin $A_4$
13β-(2′-Chlor-2′-methyl-propionylthio)-5-oximino-milbemycin $A_4$
13β-(2′,2′-Dichlorpropionylthio)-5-oximino-milbemycin $A_4$
13β-(2′,2′-Dimethylbutanoylthio)-5-oximino-milbemycin $A_4$
13β-(3′,3′-Dimethylbutanoylthio)-5-oximino-milbemycin $A_4$
13β-(2′,2′,3′,3′-Tetramethylbutanoylthio)-5-oximino-milbemycin $A_4$
13β-(p-Chlorbenzoylthio)-5-oximino-milbemycin $A_4$
13β-(3′,3′,3′-Trifluorpropionylthio)-5-oximino-milbemycin $A_4$
13β-Chloracetylthio-5-oximino-milbemycin $A_4$
13β-(2′-Chlor-3′,3′,3′-trifluorpropionylthio)-5-oximino-milbemycin $A_4$
13β-(3′,3′,3′-Trifluorpropionylthio)-5-oximino-milbemycin $A_4$
13β-(4′-Heptylcarbonylthio)-5-oximino-milbemycin $A_4$
13β-(4′-Heptylcarbonylthio)-5-oximino-milbemycin $A_3$
13β-(2′-Trifluormethylbenzoylthio)-5-oximino-milbemycin $A_4$
13β-(2′-Trifluormethylbenzoylthio)-5-oximino-milbemycin $A_3$
13β-((R/S)-2′-Phenylpropionylthio)-5-oximino-milbemycin $A_4$
13β-((R/S)-2′-Phenylpropionylthio)-5-oximino-milbemycin $A_3$
13β-(2,2′-Dimethylbutyrylthio)-5-oximino-milbemycin $A_4$
13β-(2,2′-Dimethylbutyrylthio)-5-oximino-milbemycin $A_3$
13β-(3′-Fluor-2′,2′-dimethylpropionylthio)-5-oximino-milbemycin $A_4$
13β-(3′-Fluor-2′,2′-dimethylpropionylthio)-5-oximino-milbemycin $A_3$
13β-(Methoxiacetylthio)-5-oximino-milbemycin $A_4$
13β-(Methoxiacetylthio)-5-oximino-milbemycin $A_3$
13β-(2′3′-(Difluormethylendioxid)-benzoylthio-5-oximino-milbemycin $A_4$
13β-(2′3′-(Difluormethylendioxi)-benzylthio-5-oximino-milbemycin $A_3$

Von besonderem Interesse sind Verbindungen der Formel I, in denen R für tert.Butyl oder 2-Phenylpropyl steht und $R_1$ und $R_2$ die für Formel I angegebenen Bedeutungen haben, und von diesen insbesondere diejenigen Verbindungen, in denen $R_1$ Wasserstoff und $R_2$ Methyl, Ethyl oder Isopropyl bedeutet.

Ganz besonders bevorzugt sind Verbindungen der Formel Ia, in denen R für tert.Butyl oder 2-Phenylpropyl steht und $R_1$ und $R_{2}a$ die für die Formel Ia angegebenen Bedeutungen haben, und von diesen insbesondere diejenigen Verbindungen, in denen $R_1$ Wasserstoff und $R_{2}a$ Methyl, Ethyl oder Isopropyl bedeutet.

Die vorliegende Erfindung betrifft nicht nur die Verbindungen der Formel I sondern gleichermassen ein Verfahren zu deren Herstellung. Es wurde nämlich gefunden, dass man durch Reaktion einer Verbindung der Formel II,

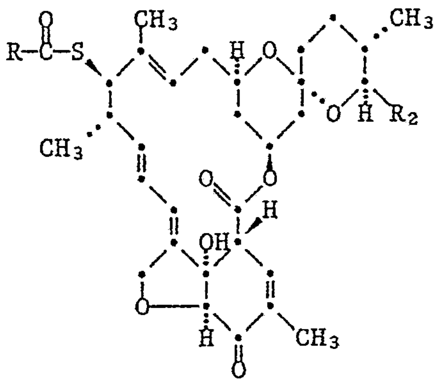

(II)

worin R und $R_2$ die für die Formel I angegebene Bedeutung haben, mit einem geeigneten Oximierungsreagenz zu den Verbindungen der Formel I gelangt.

Als geeignete Oximierungsreagenzien kommen Hydroxylamin ($NH_2OH$), Hydroxylaminderivate $NH_2OR_1$, worin $R_1$ die oben angegebene Bedeutung hat, oder davon abgeleitete Salze mit Mineralsäuren wie HCl, $NHO_3$ oder $H_2SO_4$ in Frage.

Die Reaktion wird üblicherweise in einem inerten Lösungsmittel wie Methanol, Ethanol, Tetrahydrofuran, Dioxan, Pyridin, Essigsäure, Wasser oder in Gemischen dieser Lösungsmittel bei Temperaturen von 0° bis 80°C, vorzugsweise von 20°C to 40°C, durchgeführt.

Wird Hydroxylamin in Form eines Salzes, z.B. als Hydrochlorid eingesetzt, so ist es vorteilhaft, wenn man zum Abfangen der Säure (z.B. HCl) eine der für solche Zweck üblichen Basen zusetzt und gegebenenfalls in Gegenwart eines Wasserbinders, z.B. eines Molekularsiebes, arbeitet. Als geeignete Basen kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali- und Erdalkalimetallen (CaO, BaO, NaOH, KOH, NaH, $Ca(OH)_2$, $KHCO_3$, $NaHCO_3$, $Ca(HCO_3)_2$, $K_2CO_3$, $Na_2CO_3$), sowie Alkaliace tate wie $CH_3COONa$ oder $CH_3COOK$. Darüberhinaus eignen sich auch Alkalialkoholate wie $C_2H_5ONa$, n-$C_3H_7ONa$ usw. Bevorzugt wird Triethylamin.

Im weiteren können die Verbindungen der Formel I, worin $R_1$ eine Acylgruppe bedeutet, erhalten werden, indem man die Verbindung der Formel I, worin $R_1$ für Wasserstoff steht, mit einem Säurehalogenid der Formel III

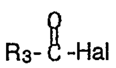 (III)

worin $R_3$ die oben angegebene Bedeutung hat und Hal Halogen, bevorzugt Chlor oder Brom bedeutet, zur Reaktion bringt.

Das Verfahren wird im allgemeinen in einem reaktionsinerten Lösungsmittel oder in einem der beteiligten Reaktanden, sofern diese flüssig sind, durchgeführt. Geeignete Lösungsmittel sind beispielsweise Diethylether, Tetrahydrofuran, Dioxan, Methylenchlorid, Tetrachlorkohlenstoff oder Chloroform. Die Reaktion wird im allgemeinen bei Temperaturen von -20° bis 100°C, vorzugsweise von 0° bis 50°C durchgeführt. Zum Abfangen der dabei als Nebenprodukt gebildeten Säuren wird die Reaktion zweckmässigerweise in Gegenwart eines Neutralisationsmittels durchgeführt.

Als solche kommen organische Basen in Betracht, beispielsweise tertiäre Amine wie Trialkylamine (Trimethylamin, Triethylamin, Diisopropylmethylamin oder Tripropylamin) 1,5-Diazabicyclo[4.3.0]non-5-en, 1,4-Diazabicylo[2.2.0]octan, 1,8-Diazabicyclo[5.4.0]undec-7-en,, Pyridin und Pyridinbasen (4-Dimethylaminopyridin oder 4-Pyrrolidylaminopyridin), bevorzugt ist Pyridin. Das Neutralisationsmittel wird üblicherweise in mindestens äquimolarer Menge in bezug auf die Ausgangsstoffe eingesetzt.

Die Verbindung der Formel II werden aus den Verbindungen der Formel IV,

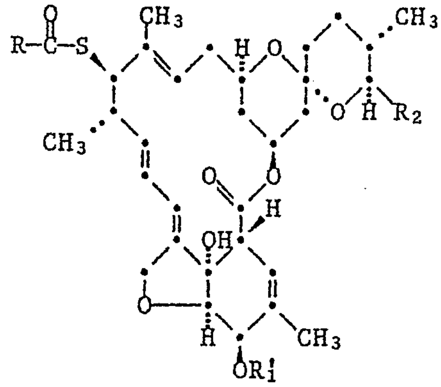

(IV)

in welcher

$R_1^{'}$ Wasserstoff bedeutet und R und $R_2$ die für die Formel I bzw. II angegebene Bedeutung haben, durch Oxidation mit einem geeigneten Reagenz erhalten. Als solche kommen z.B. aktiviertes Mangandioxid, Oxalylchlorid/Dimethylsulfoxid/Triethylamin, Chromtrioxid/Pyridin oder weitere dem Fachmann geläufige Oxidationsmittel im Betracht. Man arbeitet dabei normalerweise in einem reaktionsinerten Lösungsmittel.

Als Lösungsmittel kommen Kohlenwasserstoffe, wie Hexan, Heptan, Octan, aromatische Kohlenwasserstoffe, wie Benzol, Toluol, Xylole und bevorzugte chlorierte Kohlenwasserstoffe, inbesondere Methylenchlorid in Frage. Die Reaktionen weren bei Temperaturen von -80°C bis 60°C, vorzugsweise von -60°C bis 30°C durchgeführt.

Die Verbindungen der Formel II sind aufgrund ihrer spezifischen Struktur für die Herstellung der hochaktiven Verbindungen der Formel I prädestiniert. Sie haben somit Zwischenproduktecharakter und zeigen zum Teil ebenfalls ekto- und endoparasitizide Aktivität wie die Endprodukte. Die Verbindungen der Formel II sind daher einschliesslich ihrer Herstellungsmethoden ein Bestandteil vorliegender Erfindung.

Die vorliegende Erfindung betrifft auch Verfahren, die es erlauben, in der 13-Position von Milbemycin- oder 13-Deoxy-22,23-dihydro-Avermectinaglykon-Derivaten sowie von 23-Deoxy-Derivaten der natürlichen Antibiotika S541 eine β-Actylthio-Gruppe gezielt einzuführen und damit zu den hochwirksamen neuen Parasitiziden und Insektiziden der Formel IV zu gelangen, die gleichzeitig auch für weitere Derivatisierungen verwendet werden können.

Zur Herstellung von Thiolestern der Formel IV, worin RCOS- in der 13β-Position steht, geht man von einer Verbindung der Formel V

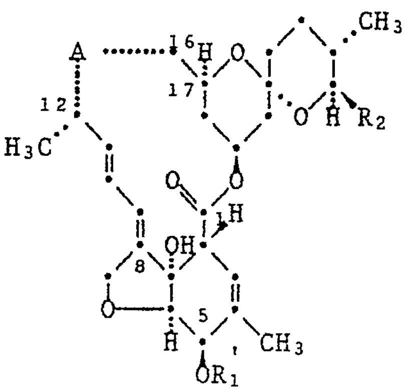

(V)

worin A für eine der Gruppe a, b oder c

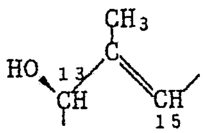 ,

(a)

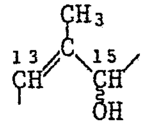 oder 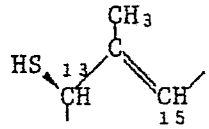

(b) (c)

steht, worin $R_1'$ Wasserstoff, eine Silyl- oder Acylgruppe bedeutet, und $R_2$ die für Formel I angegebenen Bedeutungen hat, aus. Eine Verbindung der Formel V, in welcher R eine Schutzgruppe bedeutet und $R_2$ die unter Formel I angegebenen Bedeutungen besitzt, wird mit einem zur Einführung oder zum Aufbau einer 13β-Thiolestergruppe geeigneten Reagenz behandelt. Danach kann die $R_1'$ -Schutzgruppe, sofern eine freie 5-Hydroxy-Verbindung erwünscht ist, hydrolytisch abgespalten werden.

Unter OH-Schutzgruppen für den Substituenten $R_1'$ sollen hier und im folgenden die in der organischen Chemie üblichen Schutzfuktionen verstanden werden. Dabei handelt es sich insbesondere um Acyl- und Silylgruppen. Geeignete Acylgruppen sind beispielsweise die Reste $R_4$-C(O)-, worin $R_4$ vorzugsweise für $C_1$-$C_6$-Alkyl, $C_1$-$C_6$-Haloalkyl oder unsubstituiertes oder durch Halogen, $C_1$-$C_3$-Alkyl, $CF_3$ oder Nitro substituiertes Phenyl steht. Als geeignete Silylgruppen für $R_1'$ kommt der Rest -Si($R_5$)($R_6$)($R_7$) in Frage, wobei $R_5$, $R_6$ und $R_7$ vorzugsweise unabhängig voneinander für $C_1$-$C_4$-Alkyl, Benzyl oder Phenyl stehen und beispielsweise eine der Gruppen Trimethylsilyl, tris(tert.Butyl)silyl, Diphenyl-tert.butylsilyl, bis(Isopropyl)methylsilyl, Dimethyl-(2,3-dimethyl-2-butyl)silyl, Triphenylsilyl usw. und insbesondere tert.Butyl-dimethylsilyl bildet. Die 5-OH-Gruppe kann auch als Benzylether oder Methoxiethoximethylether vorliegen.

Verbindungen der Formel V, worin A für die Gruppe a steht, werden hier und im folgenden mit Va, die Verbindungen mit der Gruppe b mit Vb und die Verbindungen mit der Gruppe c als Vc bezeichnet.

Zur Einführung der 13β-Thiolestergruppe in Verbindung der Formeln Va und Vb geeignete Reagenzien sind beispielsweise:

a) Thiocarbonsäuren der Formel VI

RCOSH (VI)

b) Thioamide der Formel VII

$RCSN(C_1\text{-}C_4\text{-Alkyl})_2$ (VII)

worin die Alkylreste vorzugsweise Methyl bedeuten.

Ein anderes Verfahren zur Herstellung der Thiolester der Formel I besteht in der Umsetzung einer Verbindung der Formel Vc mit c) einem Säurehalogenid der Formel VIII

RCOhal (VIII),

worin hal Halogen, bevorzugt Chlor oder Brom, bedeutet, oder

d) einem Säureanhydrid der Formel IX

$(RCO)_2O$ (IX),

wobei sich die Thiocarbonsäuren und Thioamide für alle Verbindungen der Formeln Va und Vb eignen, vorzugsweise aber für Verbindungen der Formel Vb verwendet werden, während Säurehalogenide und Säureanhydride bei Verbindungen der Formel Vc zum Einsatz gelangen.

In den vorstehend angegebenen Formeln VI-IX besitzt R die unter Formel I angegebenen Bedeutungen.

Die Reaktion zur Herstellung von Verbindungen der Formel IV werden vorzugsweise mit Verbindungen der Formeln Va oder Vb durchgeführt, deren reaktive 5-Hydroxy-Gruppe geschützt ist.

Verbindungen der Formel IV, worin $R_1'$ eine Schutzgruppe darstellt, lassen sich durch einfache, beispielsweise hydrolytische Abspaltung der Schutzfunktion in die hochaktiven freien 5-Hydroxy-derivate (R =H) überführen und haben somit Zwischenprodukte-Charakter. Im übrigen wird der biologische Wert dieser Verbindungen durch die Schutzgruppe nicht gemindert.

Das Verfahren zur Einführung der 13β-Thiolestergruppe wird im allgemeinen in einem reaktions-inerten Lösungsmittel oder in einem der beteiligten Reaktanden, sofern diese flüssig sind, durchgeführt. Geeignete Lösungsmittel sind beispielsweise: Ether und etherartige Verbindungen wie Dialkylether (Diethylether, Diisopropylether, tert.-Butylmethylether, Dimethoxyethan, Dioxan, Tetrahydrofuran oder Anisol); halogenierte Kohlenwasserstoffe wie Chlorbenzol, Methylenchlorid, Ethylenchlorid, Chloroform, Tetrachlorkohlenstoff oder Tetrachlorethylen; oder Sulfoxide wie Dimethylsulfoxid; ferner aromatische oder aliphatische Kohlenwasserstoffe, wie Benzol, Toluol, Xylol, Petrolether, Ligroin oder Cyclohexan. In manchen Fällen kann es von Vorteil sein, wenn die Reaktionen unter Schutzgasatmosphäre (beispielsweise Argon, Helium oder Stickstoff) und/oder in absoluten Lösungsmitteln durchgeführt werden. Gewünschtenfalls können die Endprodukte auf übliche Art und Weise gereinigt werden, beispielsweise durch Waschen, Digerieren, Extraktion, Umkristallisation oder Chromatographie.

Die Umsetzung von Verbindungen der Formel Va oder Vb mit Thiocarbonsäuren oder Thioamiden der Formeln VI bzw. VII erfolgt in Gegenwart von Orthoestern sowie in Gegenwart katalytischer Mengen einer weiteren Säure. Als dafür geeignete Säuren können Protonensäuren oder Lewis-Säuren eingesetzt werden.

Beispiele derartiger Säuren sind anorganische Säuren; Halogenwasserstoffsäure wie Chlorwasserstoffsäure, Bromwasserstoffsäure oder Jodwasserstoffsäure, Perchlorsäure sowie Schwefelsäure und organische Säuren wie Essigsäure, Trifluoressigsäure, Trichloressigsäure, Propionsäure, Oxalsäure, Ameisensäure, Benzolsulfonsäure, p-Toluolsulfonsäure oder Methansulfonsäure sowie Lewis-Säuren, wie $BF_3$, $AlCl_3$ oder $ZnCl_3$. Besonders bevorzugt sind p-Toluolsulfonsäure (auch als TsOH bezeichnet) und Schwefelsäure.

Die bei dieser Reaktion benötigten Orthoester haben die Formel X

$R_8C(OR_9)_3$ (X),

worin $R_8$ Wasserstoff oder $C_1$-$C_4$-Alkyl, bevorzugt Methyl, und $R_9$ $C_1$-$C_4$-Alkyl, bevorzugt Methyl oder Ethyl bedeuten.

Bei Verwendung von Thiocarbonsäuren oder Thioamiden der Formeln VI bzw. VII zur Herstellung von Verbindungen der Formel IV liegen die Reaktionstemperaturen im allgemeinen im Bereich von 0° bis 150°C, bevorzugt von 20° bis 130°C.

Die Reaktion von Verbindungen der Formel Vc mit Säurehalogeniden oder Säureanhydriden der Formeln VIII bzw. IX wird normalerweise in den obengenannten reaktionsinerten Lösungsmitteln im allgemeinen bei Temperaturen von -20° bis 100°C, vorzugsweise von 0° bis 70°C, durchgeführt. Zum Abfangen der dabei als Nebenprodukt gebildeten Säuren findet die Reaktion zweckmässigerweise in Gegenwart eines der oben erwähnten Neutralisationsmittel, bevorzugt Pyridin, statt.

Bei der Reaktion von Verbindungen der Formel Vb mit Thiocarbonsäuren oder Thioamiden der Formeln VI bzw. VII in Gegenwart von Orthoestern der Formel X sowie einer katalytisch wirksamen Säure können neben den Verbindungen der Formel IV als Nebenprodukte auch Verbindungen der Formel XI

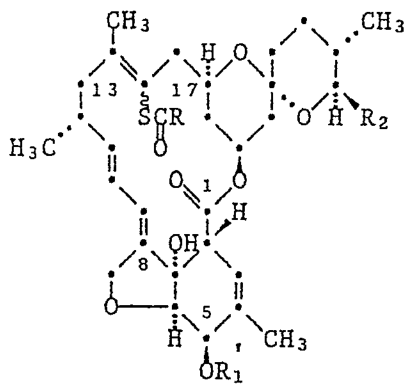

(XI)

worin $R_1'$, $R_2$ und R die unter Formel IV angegebenen Bedeutungen besitzen, gebildet werden.

Die erhaltenen Reaktionsprodukte lassen sich durch übliche Trennungsmethoden, wie beispielsweise durch fraktionierte Kristallisation oder durch Chromatographie, voneinander trennen. Unter Chromatographie werden Säulen-, Dickschicht- oder Dünnschicht-Chromatographie sowie bevorzugt Hochdruck-Flüssigkeits-Chromatographie an mineralischen Trägermaterialien wie Silicagel oder an organischen Austauscherharzen verstanden.

Die Verbindungen der Formeln Va und Vb, worin $R_2$ für Methyl, Ethyl, Isopropyl oder sek.Butyl steht und $R_1'$ Wasserstoff, eine Silyl-oder eine Acylgruppe bedeutet, sind in den Europ. Offenlegungsschriften EP 180 539 bzw. EP 147 852 beschrieben.

Verbindungen der Formel $V_a$ und $V_b$, worin $R_2$ für die Gruppe $-\underset{CH_3}{C}=CH-X$ steht und X Methyl, Ethyl oder Isopropyl bedeutet und R

Wasserstoff, eine Silyl- oder eine Acylgruppe bedeutet, lassen sich analog zu den an sich bekannten Verfahren aus den natürlich vorkommenden Antibiotika S541 herstellen, die aus der DE 35 32 794 bekannt und durch die folgende chemische Struktur charakterisiert sind:

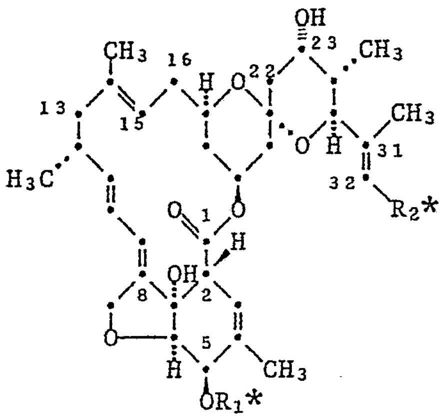

(Antibiotika S541)

| | | |
|---|---|---|
| Faktor A | $R_2^*=isoC_3H_7$ | $R_1^*=H$ |
| Faktor B | $R_2^*=CH_3$ | $R_1^*=CH_3$ |
| Faktor C | $R_2^*=CH_3$ | $R_1^*=H$ |
| Faktor D | $R_2^*=C_2H_5$ | $R_1^*=H$ |
| Faktor E | $R_2^*=C_2H_5$ | $R_1^*=CH_3$ |
| Faktor F | $R_2^*=isoC_3H_7$ | $R_1^*=CH_3$ |

Je nach Faktor werden im folgenden zur Vereinfachung der Benennung die Abkömmlinge von Antibiotika S541 als Derivate von S541A, S541B, S541C, S541D, S541E oder S541F klassifiziert.

Die in 23-Position befindliche Hydroxygruppe in den Antibiotika S541 lässt sich analog zu der in US-PS 4,328,335 bekanntgewordenen Methode entfernen und die Antibiotika S541 lassen sich so in die entsprechenden 23-Deoxi-Derivate überführen. Dabei müssen diejenigen Verbindungen mit einer freien 5-OH-Gruppe ($R_1^*=H$) zunächst selektiv geschützt werden durch Reaktion mit einem der zuvor genannten Silylierungsreagenzien Y-Si($R_5$)($R_6$)($R_7$) bzw. mit tert.-Butyldimethylsilyloxiacetylchlorid. Die Umsetzung dieser geschützten Verbindungen, in denen $R_1^*$ durch Si($R_5$)($R_6$)($R_7$) bzw. $C(=O)CH_2OSi(CH_3)_2t\text{-}C_4H_9$ ersetzt und das 23-C-Atom durch OH substituiert ist, mit p-Methylphenyl-chlorthionoformiat ergibt Derivate dei Antibiotika S541, welche in Position 23 durch $p\text{-}CH_3\text{-}C_6H_4\text{-}O\text{-}C(=S)\text{-}O\text{-}$ substituiert sind. Diese 23-O-(4-Methyl-phenoxi)-thiocarbonyl-derivate von Antibiotika S541 werden dann als Ausgangsmaterialien für die Reduktion mit Tributylzinnhydrid in Toluol in Gegenwart von Azobisisobutyronitril bei 80-120°C zu den entsprechenden 23-Deoxi-Derivaten (Position 23 unsubstituiert) eingesetzt.

Die Herstellung von Verbindungen der Formel Vc kann durch Umsetzung einer Verbindung der Formel Vb mit einem Halothionoformiat der Formel (XII)

$$Hal\text{-}\overset{\overset{\displaystyle S}{\|}}{C}\text{-}OR_{10} \qquad (XII),$$

worin $R_{10}$ für $C_2$-$C_{10}$-Haloalkyl steht, und anschliessende Reduktion des erhaltenen Produkts erfolgen.

Die Reaktion von Verbindungen der Formel Vb mit Halthionoformiaten der Formel XII erfolgt üblicherweise in den oben (bei der Einführung der 13β-Thiolestergruppe) genannten reaktionsinerten Lösungsmitteln oder in dem Halothionoformiat der Formel XII selbst. Man arbeitet zweckmässigerweise in Gegenwart eines Kondensationsmittels. Als solche kommen organische und anorganische Basen in Betracht, z.B. tertiäre Amine wie Trialkylamine (Trimethylamin, Triethylamin, Tripropylamin, usw.), Pyridin und Pyridinbasen (4-Dimethylaminopyridin, 4-Pyrrolidylaminopyridin usw.), bevorzugt ist Pyridin. Das Kondensationsmittel wird üblicherweise in mindestens äquimolarer Menge in Bezug auf die Ausgangsstoffe eingesetzt. Die Reaktionstemperaturen liegen bei dieser Umsetzung im allgemeinen bei -50° bis +150°C, vorzugsweise bei -20° bis +100°C. Die bei dieser Reaktion entstehenden Thiolcarbonate der Formel IV ($R = OR_{10}$) lassen sich durch einfache Reduktion, z.B. mit Zink in Eisessig, in die 13β-Mercapto-Verbindungen der Formel Vc überführen. Diese Reduktion erfolgt zweckmässigerweise in einem üblichen, reaktionsinerten, organischen Lösungsmittel (beispielsweise wie vorstehend angegeben) bei Temperaturen zwischen 0° und 50°C, vorzugsweise 20° und 50°C.

Durch Acylierung oder Silylierung der 5-OH-Gruppe werden alle jene Derivate der Formeln IV, Va, Vb und Vc hergestellt, bei denen $R_1'$ eine andere Bedeutung als Wasserstoff ($R_1'$ = OH-Schutzgruppe) hat. Die Einführung der Acylgruppe erfolgt üblicherweise mit den entsprechenden Acylhalogeniden oder Acylanhydriden und wird vorzugsweise zur Einführung der eingangs definierten $R_4C(O)$- Gruppe benutzt. Zur Silylierung verwendet man zweckmässigerweise ein Silan der Formel Y-Si($R_5$)($R_6$)($R_7$), worin $R_5$, $R_6$, $R_7$ einen der eingangs genannten Reste darstellen, wobei der Begriff Acylhalogenid für Acylchlorid oder Acylbromid steht

und wobei Y eine Silylabgangsgruppe bedeutet. Zu den Silylabgangsgruppen Y zählen beispielsweise Bromid, Chlorid, Cyanid, Azid, Acetamid, Trifluoracetat oder Trifluormethansulfonat. Diese Aufzählung stellt keine Limitierung dar, der Fachmann kennt weitere typische Silylabgangsgruppen.

5-O-Acylierungen und 5-O-Silylierungen werden in wasserfreiem Milieu, vorzugsweise in inerte Lösungsmitteln und besonders bevorzugt in aprotischen Lösungsmitteln durchgeführt. Die Reaktion läuft vorteilhaft im Temperaturbereich von 0° bis +80°C, bevorzugt bei +10° bis +40°C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage.

Die Entfernung dieser Silyl- und Acylreste $R_1$ in der 5-Position geschieht durch selektive milde Hydrolyse ($\rightarrow R_1$ =H) beispielsweise mit Arylsulfonsäure in alkoholischer Lösung oder nach einer anderen dem Fachmann geläufigen Methode.

Das beschriebene Verfahren zur Herstellung der Verbindungen der Formel I ist in allen Teilschritten ein Bestandteil vorliegender Erfindung.

Besonders bevorzugt im Rahmen dieser Erfindung ist ein Verfahren zur Herstellung von Verbindungen der Formel Ia.

Die Verbindungen der Formel I eignen sich ausgezeichnet zur Bekämpfung von Schädlingen an Tieren und Pflanzen in allen Entwicklungsstadien, darunter insbesondere von tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophiliae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Coccidae, Diaspididae und Eriophydidae (z.B. die Rostmilbe aud Citrusfrüchten): der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getriede, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Gattungen Meloidogyne, Heterodera, Pratylenchus, Ditylenchus, Radopholus, Rhizoglyphus und andere.

Ferner sind die Verbindungen gegen Helminthen in allen Entwicklungsstadien wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Anyclostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Gattungen Nematodirus, Cooperia und Oesophagostomum greifen den Intestinaltrakt des Wirtstiers an, während andere der Gattungen Haemonchus und Ostertagia im Magen und solche der Gattung Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Weiter sind die Verbindungen der Formel I zur Bekämpfung von humanpathogenen Parasiten geeignet, unter denen als typsiche, im Verdauungstrakt vorkommende Vertreter solche der Gattungen Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Gattungen Wuchereria, Brugia, Onchocerca und Loa aus der Familie der Filariidae, die im Blut, im Gewebe und verschiedenen Organen vorkommen, ferner gegen Dracunculus und Parasiten der Gattungen Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie

die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht dosiert. Ueber geschlossenen Kultur-Anbauflächen werden sie in Mengen von 10 g bis 1000 g pro Hektar angewendet. Sie kommen ferner in Pferchen, Gehegen, Stallungen oder sonstigen Räumen zur Anwendung.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberfluachenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorganulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstofes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufier werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eine Naphthalinsulfonsäure-Formaldehydkondensationsproduktes.

Ferner kommen auch entsprechend Phosphate wie z.B. Salze des Phosphorsäureesters eines p-Nonylphenol-(4-14)-Aethylenoxyd-Adduktes oder Phospholipide in Frage.

Die in der Formulierungstechnik gebräuchlichen Tenside sind u.a. in folgender Publikation beschrieben:
"1986 International McCutcheon's Emulsifiers and Detergents"
The Manufacturing Confectioner Publishing Co.,
Glen Rock, New Jersey, USA.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als Wirkstoff mindestens eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### 1. Herstellung von Ausgangs- und Zwischenprodukten

### Beispiel H1: Herstellung von 13β-Mercapto-milbemycin D und von 5-O-tert.Butyldimethylsilyl-13β-mercapto-milbemycin D

a) Zu einer Lösung von 209 mg (0,305 mmol) 5-O-tert.Butyldimethylsilyl-15-hydroxy$\Delta^{13,14}$-milbemycin D und 0,012 ml (120 mg, 1,52 mmol) Pyridin in 3 ml Dichlormethan werden unter Argon bei -10° C unter Rühren 0,1 ml (157 mg, 0,689 mmol) 2,2,2-Trichlorethylchlorthionoformiat zugetropft. Nach 1 Stunde Rühren bei Raumtemperatur wird mit 5 %iger wässriger $NaHCo_3$-Lösung und Diethylether aufgearbeitet. Die Chromatographie des Rohprodukts [20 g Kieselgel/Laufmittel:Ethylacetat/Hexan (1:4)] liefert 282 mg z.T noch verunreinigtes 5-O-tert.Butyldimethylsilyl-13β-2,2,2-trichlorethoxicarbonylthio-milbemycin D.

Eine Suspension von 320 mg (4,9 mmol) Zink-Pulver in einer Lösung von 227 mg dieses Rohproduktes in 0,5 ml Diethylether, 2 ml 90 %iger wässriger Essigsäure und 3 Tropfen HCl (1M) werden bei Raumtempera tur 16 Stunden unter Argon gerührt. Das Gemisch wird mit Diethylether verdünnt, durch Celite filtriert, mit $MgSO_4$ getrocknet und eingeengt. Die Chromatographie des Rohproduktes [20 g Kieselgel/Laufmittel:Ethylcetat/Hexan (12:88)] ergibt 72 mg (40 %) 5-O-tert.Butyldimethylsilyl-13β-mercapto-milbemycin D.

b) Dieses gereinigte Produkt wird mit 2 ml einer 1 %igen Lösung von p-Toluolsulfonsäure in Methanol 2 Stunden bei Raumtemperatur gerührt. Nach der Aufarbeitung mit 5 %iger wässriger $NaHCO_3$-Lösung und Diethylether wird das Rohprodukt [20 g Kieselgel/Läufmittel:-Ethylacetat/Hexan (2:3)] chromatographiert. Man erhält 54 mg (89 %) 13β-Mercapto-milbemycin D mit folgenden spektroskopischen Daten:

$^1$H-NMR (300 MHz: $CDCl_3$: TMS)
1,61 ppm (s) ($C_{14}CH_3$)
1,87 ppm (s) ($C_4CH_3$)
3,31 ppm (dd; J = 5,4 und 10,9), ($C_{13}H$)
Massenspektrum m/e: 588 ($M^+$, $C_{33}H_{48}O_7S$) 460, 309, 277, 209, 181.

Beispiel H2: Herstellung von 5-O-tert.Butyldimethylsily-13β-Trichlorethoxicarbonylthiomilbemycin $A_4$

Zu einer Lösung von 100 mg (0,15 mmol) 5-O-tert.Butyldimethylsilyl-15-hydroxi-$\Delta^{13,14}$-milbemycin $A_4$ und 0,060 ml (59 mg, 0,75 mmol) Pyridin in 3 ml Dichlormethan werden unter Argon bei -10° C unter Rühren 0,13 ml (205 mg, 0,9 mmol) 2,2,2-Trichlorethylchlorthionoformiat zugetropft. Nach 30 Min. Rühren bei Raumtemperatur wird mit 5%iger wässriger $NaHCO_3$-Lösung und Diethylether aufgearbeitet. Die Chromatographie des Rohproduktes [20 g Kieselgel/Laufmittel: Ethylacetat/Hexan (1:12)] liefert 40 mg 5-O-tert.Butyldimethylsilyl-13β-2,2,2-trichlorethoxicarbonylthiomilbemycin $A_4$.

$^1$H-NMR (300 MHz; $CDCl_3$; TMS)
3,82 ppm (d, J = 10 Hz) ($C_{13}H$)
4,75 ppm (d, J = 14 Hz) und 4,86 (d) (J = 14 Hz) ($Cl_3CCH_2$)
Massenspektrum (FD) m/e: 862 ($M^+$, $C_{41}H_{61}Cl_3O_9SSi$)

Beispiel H3: Herstellung von 5-O-tert.Butyldimethylsily-13β-mercapto-milbemycin $A_4$

Eine Lösung von 2,9 g (3,36 mmol) 5-O-tert.Butyldimethylsilyl-13β-2,2,2-trichlorethoxicarbonylthio-milbemycin $A_4$ in 40 ml Tetrahydrofuran wird mit 1,05 g (16,1 mmol) Zink und 20 ml gesättigtem wässrigem $NH_4Cl$ während 5 Stunden schnell gerührt. Aufgearbeitet wird mit Wasser und Diethylether. Die Chromatographie des Rohprodukts [Kieselgel/Laufmittel: Ethylacetat/Hexan (1:9)] liefert 2,46 g 5-O-tert.Butyldimethylsilyl-13β-mercapto-milbemycin $A_4$.

$^1$H-NMR (300 MHz; $CDCl_3$; TMS)
3,29 (ppm (dd; J = 10 und 5 Hz) ($C_{13}H$)
Massenspektrum (FD) m/e: 688 ($M^+$, $C_{38}H_{60}O_7SSi$)

Beispiel H4: Herstellung von 5-O-tert.Butyldimethylsilyl-13β-(4'-heptylcarbonylthio)-milbemycin $A_4$ und von 13β-(4'-Heptylcarbonylthio)-milbemycin $A_4$

a) Zu einer Lösung von 110 mg (0,154 mmol) 5-O-tert.Butyldimethylsilyl-13β-mercapto-milbemycin $A_4$ in 5 ml abs. Chloroform und 2 ml Pyridin werden unter Argon bei 0° C unter Rühren 0,2 ml 4-Heptylcarbonsäurechlorid zugegeben. Nach 7 Stunden Rühren bei Raumtemperatur wird mit eiskalter, verdünnter wässriger HCl-, verdünnter wässriger $NaHCO_3$-Lösung und Diethylether aufgearbeitet. Die Chromatographie des Rohprodukts [10 g Kieselgel/Laufmittel: Ethylacetat/Hexan (1:5)] liefert 92 mg 5-O-tert.Butyldimethylsilyl-13β-(4'-heptylcarbonylthio)-milbemycin $A_4$.

b) Dieses gereinigte Produkt wird mit 1 ml einer 1%igen Lösung von p-Toluolsulfonsäure in Methanol 3 Stunden bei Raumtemperatur gerührt. Nach der Aufarbeitung mit 5%iger wässriger $NaHCO_3$-Lösung und Diethylether wird das Rohprodukt chromatographiert [10 g Kieselgel/Laufmittel: Ethylacetat/Hexan (1:3)], wobei 49 mg 13β-(4'-Heptylcarbonylthio)-milbemycin $A_4$ mit folgenden spektroskopischen Daten erhalten:

$^1$H-NMR (300 MHz; $CDCl_3$; TMS)
3,95 ppm (d, J = 6 Hz) ($C_6H$)
3,97 ppm (d, J = 10 Hz) ($C_{13}H$)
MS (FD) m/e: 700 ($M^+$, $C_{40}H_{60}O_8S$)

Analog zu Beispiel H4 werden folgende Verbindungen hergestellt:

Beispiel H5: 13β-Acetylthio-milbemycin $A_4$

MS (FD) m/e: 616 ($M^+$, $C_{34}H_{48}O_8S$)

Beispiel H6: 13β-(2′-Trifluormethylbenzolythio)-milbemycin $A_4$

$^1H$-NMR (300 MHz; $CDCl_3$; TMS)
3,97 ppm (d, J = 6 Hz) ($C_6H$)
4,21 ppm (d, J = 10 Hz) ($C_{13}H$)
7,48-7,76 ppm (m) (4 aromat. H)
MS (FD) m/e: 746 ($M^+$, $C_{40}H_{49}O_9SF_3$)

Beispiel H7: 13β-((R/S)-2′-Phenylpropionylthio)-milbemycin $A_4$; Diastereomerengemisch

$^1H$-NMR (300 MHz; $CDCl_3$; TMS)
3,75 und 3,83 ppm (2 q, J = 6 Hz) ($C'_2$,H)
3,82 und 3,93 ppm (2 d, J = 6 Hz) ($C_6H$)
3,81 und 3,94 ppm (2 d, J = 10 Hz) ($C_{13}H$)
7,11-7,37 ppm (m) (5 aromat. H)

Beispiel H8: 13β-(2′,2′-Dimethylbutyrylthio)-milbemycin $A_4$

$^1H$-NMR (300 MHz; $CDCl_3$; TMS)
3,91 ppm (d, J = 10 Hz) ($C_{13}H$)
3,95 ppm (d, J = 6 Hz) ($C_6H$)
MS (FD) m/e: 672 ($M^+$, $C_{38}H_{56}O_8S$)

Beispiel H9: 13β-(3′-Chlor-2′,2′-dimethylpropionylthio)-milbemycin $A_4$

$^1H$-NMR (300 MHz; $CDCl_3$; TMS)
3,93 ppm (d, J = 10 Hz) ($C_{13}H$)
3,96 ppm (d, J = 6 Hz) ($C_6H$)
3,60 ppm (AB-System, J = 13 Hz; A-Teil; 3,57 ppm, B-Teil: 3,63 ppm) ($CH_2Cl$)

Beispiel H10: 13β-(2′-Methyl-2′-phenylpropionylthio)-milbemycin $A_4$

$^1H$-NMR (300 MHz; $CDCl_3$; TMS)
3,89 ppm (d, J = 11 Hz) ($C_{13}H$)
3,96 ppm (d, J = 6 Hz) ($C_6H$)
7,19-7,37 ppm (m) (5 aromat. H)

Beispiel H11: 13β-(3′-Fluor-2′,2′-dimethylpropionylthio)-milbemycin $A_4$

$^1H$-NMR (300 MHz; $CDCl_3$; TMS)
3,94 ppm (d, J = 6 Hz) ($C_6H$)
3,98 ppm (d, J = 10 Hz) ($C_{13}H$)
4,37 ppm (d, J = 47 Hz) ($CH_2F$)
MS (FD) m/e: 676 ($M^+$, $C_{37}H_{53}FO_8S$)

Beispiel H12: 13β-Methoxiacetylthio-milbemycin $A_4$

$^1H$-NMR (300 MHz; $CDCl_3$; TMS)
3,94 ppm (d, J = 6 Hz) ($C_6H$)
4,03 ppm (d, J = 10 Hz) ($C_{13}H$)
3,45 ppm (s) ($\underline{CH_3}OCH_2$)
4,04 ppm (s) ($CH_3O\underline{CH_2}$
MS (FD) m/e: 646 ($M^+$, $C_{35}H_{50}O_9S$)

Beispiel H13: 13β-((S)-2′-Phenylpropionylthio)-milbemycin $A_4$

$^1H$-NMR (300 MHz; $CDCl_3$; TMS)
3,83 ppm (q, J = 6 Hz) ($C_2$,H)
3,93 ppm (d, J = 6 Hz) ($C_6H$)
3,94 ppm (d, J = 10 Hz) ($C_{13}H$)
7,18-7,37 ppm (m) (5 aromat. H)

Beispiel H14: 13β-((R)-2′-Phenylpropionylthio)-milbenycin $A_4$

$^1H$-NMR (300 MHz; $CDCl_3$; TMS)
3,75 ppm (q, J = 6 Hz) ($C_2$,H)
3,81 ppm (d, J = 10 Hz) ($C_{13}H$)
3,82 ppm (d, J = 6 Hz) ($C_6H$)
7,11-7,26 ppm (m) (5 aromat. H)

Beispiel H15: Herstellung von 13β-Pivaloylthio-milbemycin $A_4$

Eine Lösung von 280 mg (0,416 mmol) 5-O-tert.-Butyldimethylsily-15-hydroxy-$\Delta^{13,14}$-milbemycin $A_4$, 0,4 ml Orthoessigsäure-trimethylester und 0,5 ml Thiopivalinsäure in 4 ml Toluol wird während 6 Stunden auf 60°C erhitzt. Aufgearbeitet wird mit Diethylether und 5%iger wässriger $NaHCO_3$-Lösung. Die Chromatographie an

Kieselgel (Ethylacetat/Hexan 1:6) liefert 61 mg 5-O-tert.-Butyldimethylsilyl-13β-pivaloylthio-milbemycin $A_4$.

Dieses Material wird mit 2 ml einer 40%igen wässrigen Lösung von HF und Acetonitril (5:95) während 2 Stunden bei Raumtemperatur behandelt. Die Aufarbeitung in Diethylether mit 5%iger wässriger $NaHCO_3$-Lösung und Chromatographie an Kieselgel (Ethylacetat/Hexan 1:2) liefert 21 mg 13β-Pivaloylthio-milbemycin $A_4$.

1H-NMR (250 MHz; $CDCl_3$; TMS):
1,25 ppm (s)[$(CH_3C]$
3,97 ppm (d)(J = 10 Hz)($C_{13}H$)
4,01 ppm (d)(J = 6 Hz)($C_6H$)

Beispiel H16: Herstellung von 13β-Acetylthio-milbemycin $A_4$

Analog Beispiel H16 wird 13βAcetylthio-milbemycin $A_4$ hergestellt:
$^1$H-NMR (300 MHz; $CDCl_3$; TMS)
2,32 ppm (s)($CH_3COS$)
3,96 ppm (d,J = 6 Hz)($C_6H$)
4,03 ppm (d,J = 10 Hz)($C_{13}H$).

## 2. Herstellung von Endprodukten der Formel I

Beispiel E1: Herstellung von 13β-Pivaloylthio-5-oximino-milbemycin $A_4$

a) Eine Lösung von 49 mg (0,075 mmol) 13β-Pivaloylthio-milbemycin $A_4$ in 2 ml Dichlormethan wird mit 98 mg Mangandioxid während 30 Minuten bei Raumtemperatur stark gerührt. Das Mangandioxid wird durch Celite abfiltriert und nach dem Einengen der Lösung erhält man rohes 13β-Pivaloylthio-5-keto-milbemycin.

b) Dieses Rohprodukt wird zusammen mit 8 mg (0,12 mmol) Hydroxylaminhydrochlorid in 1 ml Pyridin gelöst. Nach 30 Minuten Rühren bei RT wird mit Diethylether und 2M wässriger HCl aufgearbeitet. Die Chromatographie des Rohprodukts [20 g Kieselgel/Laufmittel: Ethylacetat/Hexan (1:4)] liefert 15 mg (29 %) 13β-Pivaloylthio-5-oximino-milbemycin $A_4$.

$^1$H-NMR (300 MHz; $CDCl_3$; TMS)
3,36 ppm (m) ($C_2H$)
3,93 ppm (d; J = 10) ($C_{13}H$)
5,77 ppm (s) ($C_3H$)
Massenspektrum (FD) m/e: 671 ($M^+$, $C_{37}H_{54}NO_8S$)

Beispiel E2: Herstellung von 13β-(4′-Heptylcarbonylthio)-5-oximino-milbemycin $A_4$

a) Zu einer Lösung von 30 mg (0,043 mmol) 13β-(4′-Heptylcarbonylthio)-milbemycin $A_4$ in 10 ml abs. Methylenchlorid werden 128 mg (1,47 mmol) aktiviertes Mangandioxid gegeben. Das Gemisch wird 20 Min. bei Raumtemperatur heftig gerührt, durch Hyflo filtriert und das Filtrat eingeengt. Dabei werden 27 mg 13β-(4′-Heptyl-carbonylthio)-5-oxo-milbemycin $A_4$ erhalten.

b) Dieses Material wird in 0,5 ml Dioxan und 0,5 ml Methanol gelöst und es werden 35 mg (0,05 mmol) Hydroxylaminhydrochlorid und 1 Tropfen Wasser zugegeben. Das Gemisch wird 8 Std. bei Raumtemperatur gerührt. Nach der Aufarbeitung mit 5%iger wässriger $NaHCO_3$-Lösung und Diethylether wird das Rohprodukt chromatographiert [20 g Kieselgel/Laufmittel Ethylacetat/Hexan (1:3)], wobei 10 mg 13β-(4′-Heptylcarbonylthio)-5-oximino-milbemycin $A_4$ mit folgenden spektroskopischen Daten anfallen:

$^1$H-NMR (300 MHz; $CDCl_3$; TMS)
3,97 ppm (d, J = 10 Hz) ($C_{13}H$)
4,66 ppm (s) ($C_6H$)
7,55 ppm (s) (N-OH)
MS (FD) m/e: 713 ($M^+$, $C_{40}H_{59}NO_8S$)

Analog zu den Beispielen E1 und E2 werden auch folgende Verbindungen hergestellt:

Beispiel E3: 13β-(2′-Trifluormethylbenzoylthio)-5-oximino-milbemycin $A_4$

$^1$H-NMR (300 MHz; $CDCl_3$; TMS)
4,67 ppm (s) ($C_6H$)
4,20 ppm (d, J = 10 Hz) ($C_{13}H$)
7,52-7,78 ppm (m) (4 aromat. H)
MS (FD) m/e: 759 ($M^+$, $C_{40}H_{49}NO_8SF_3O$)

Beispiel E4: 13β-(3′-Fluor-2′,2′-dimethylpropionylthio)-5-oximino-milbemycin $A_4$

3,98 ppm (d, J = 10 Hz) ($C_{13}H$)
4,37 ppm (d, J = 47 Hz) ($CH_2F$)
4,65 ppm (s) ($C_6H$)
8,00 ppm (s) (N-OH)
MS (FD) m/e: 689 ($M^+$, $C_{37}H_{52}NO_8SF$)

Beispiel E5: 13β-((R/S)-2'-Phenylpropionylthio)-5-oximino-milbemycin $A_4$; Diastereomerengemisch
$^1$H-NMR (300 MHz; $CDCl_3$; TMS)
3,83 und 3,85 ppm (2q, J = 6 Hz), ($C'_2$,H)
4,63 und 4,64 ppm (2s, ($C_6$H)
3,91 und 3,94 ppm (2, J = 10 Hz) ($C_{13}$H)
7,18-7,36 ppm (m) (5 aromat. H)

Beispiel E6: 13β-(2',2'-Dimethylbutyrlthio)-5-oximino-milbemycin $A_4$
$^1$H-NMR (300 MHz; $CDCl_3$; TMS)
3,92 ppm (d, J = 10 Hz) ($C_{13}$H)
4,65 ppm (s) ($C_6$H)
7,75-8,20 ppm (m) (N-OH)
MS (FD) m/e: 658 ($M^+$, $C_{38}H_{55}NO_8S$)

Beispiel E7: 13β-(3'-Chlor-2',2'-dimethylpropionylthio)-5-oximino-milbemycin $A_4$
$^1$H-NMR (300 MHz; $CDCl_3$; TMS)
3,59 ppm (AB-System, J = 12 Hz, A-Teil 3,56 ppm, B-Teil 3,62 ppm) ($CH_2Cl$)
4,98 ppm (d, J = 10 Hz) ($C_{13}$H)
4,65 ppm (s) ($C_6$H)
7,87 ppm (s) (N-OH)
MS (FD) m/e: 705 ($M^+$, $C_{37}H_{52}ClNO_8S$)

Beispiel E8: 13β-((S)-2'-Phenylpropionylthio)-5-oximino-milbemycin $A_4$
$^1$H-NMR (300 MHz; $CDCl_3$; TMS)
3,83 ppm (q, J = 6 Hz) ($C'_2$,H)
3,93 ppm (d, J = 10 Hz) ($C_{13}$H)
4,64 ppm (s) ($C_6$H)
7,20-7,36 ppm (m) (5 aromat. H)
7,81 ppm (s) (N-OH)
MS (FD) m/e: 719 ($M^+$, $C_{41}H_{53}NO_8S$)

Beispiel E9: 13β-((R)-2'-Phenylpropionylthio)-5-oximino-milbemycin $A_4$
$^1$H-NMR (300 MHz; $CDCl_3$; TMS)
3,85 ppm (q, J = 6 Hz) ($C'_2$,H)
3,92 ppm (d, J = 10 Hz) ($C_{13}$H)
4,63 ppm (s) ($C_6$H)
7,18-7,35 ppm (m) (5 aromat. H)
7,91 ppm (s) (N-OH)
MS (FD) m/e: 719 ($M^+$, $C_{41}H_{53}NO_8S$)

Beispiel E10: 13β-(2',3'-(Difluormethylendioxi)-benzoylthio)-5-oximino-milbemycin $A_4$
$^1$H-NMR (300 MHz; $CDCl_3$; TMS)
3,36 ppm (m) ($C_2$H)
4,66 ppm (s) ($C_6$H)
4,27 ppm (d, J = 10 Hz) ($C_{13}$H)
7,09 ppm - 7,63 ppm (m) (3 aromat. H)
7,94 ppm (s) (N-0H)
MS (FD) m/e: 771 ($M^+$, $C_{40}H_{47}F_2NO_{10}$)

Analog zu den beschriebenen Arbeitsweisen werden auch die nachfolgend zusammen mit Verbindungen der vorgängigen Beispiele genannten Verbindungen der Formel I hergestellt:

Tabelle 1

Typische Vertreter von Verbindungen der Formel I, worin $R_1$ Wasserstoff ist.

| Verb. Nr. | $R_2$ | R |
|---|---|---|
| 1.1 | $CH_3$ | H |
| 1.2 | $C_2H_5$ | H |
| 1.3 | $C_3H_7$-iso | H |
| 1.4 | $C_4H_9$-sek | H |
| 1.5 | $CH_3$ | $CH_3$ |
| 1.6 | $C_2H_5$ | $CH_3$ |
| 1.7 | $C_3H_7$-iso | $CH_3$ |
| 1.8 | $C_4H_9$-sek | $CH_3$ |
| 1.9 | $CH_3$ | $C(CH_3)_3$ |
| 1.10 | $C_2H_5$ | $C(CH_3)_3$ |
| 1.11 | $C_3H_7$-iso | $C(CH_3)_3$ |
| 1.12 | $C_4H_9$-sek | $C(CH_3)_3$ |
| 1.13 | $CH_3$ | $CH_3OCH_2$ |
| 1.14 | $C_2H_5$ | $CH_3OCH_2$ |
| 1.15 | $C_3H_7$-iso | $CH_3OCH_2$ |
| 1.16 | $C_4H_9$-sek | $CH_3OCH_2$ |
| 1.17 | $CH_3$ | $CH_3OC(CH_3)_2$ |
| 1.18 | $C_2H_5$ | $CH_3OC(CH_3)_2$ |
| 1.19 | $C_3H_7$-iso | $CH_3OC(CH_3)_2$ |
| 1.20 | $C_4H_9$-sek | $CH_3OC(CH_3)_2$ |
| 1.21 | $CH_3$ | $(CH_3)_2CH$ |
| 1.22 | $C_2H_5$ | $(CH_3)_2CH$ |
| 1.23 | $C_3H_7$-iso | $(CH_3)_2CH$ |
| 1.24 | $C_4H_9$-sek | $(CH_3)_2CH$ |
| 1.25 | $CH_3$ | $CCl_3$ |
| 1.26 | $C_2H_5$ | $CCl_3$ |
| 1.27 | $C_3H_7$-iso | $CCl_3$ |
| 1.28 | $C_4H_9$-sek | $CCl_3$ |
| 1.29 | $CH_3$ | $CF_3$ |
| 1.30 | $C_2H_5$ | $CF_3$ |
| 1.31 | $C_3H_7$-iso | $CF_3CHCl$ |
| 1.32 | $C_4H_9$-sek | $CF_3$ |
| 1.33 | $CH_3$ | $C(Cl_3)CHCl$ |
| 1.34 | $C_2H_5$ | $C(Cl_3)CHCl$ |
| 1.35 | $C_3H_7$-iso | $CF_3CH_2$ |
| 1.36 | $C_4H_9$-sek | $C(Cl_3)CHCl$ |
| 1.37 | $CH_3$ | $ClCH_2CH_2CH_2$ |
| 1.38 | $C_2H_5$ | $ClCH_2CH_2CH_2$ |

Tabelle (Fortsetzung)

| Verb. Nr. | $R_2$ | R |
|---|---|---|
| 1.39 | $C_3H_7$-iso | $ClCH_2CH_2CH_2$ |
| 1.40 | $C_4H_9$-sek | $ClCH_2CH_2CH_2$ |
| 1.41 | $CH_3$ | $CH_2$=CH |
| 1.42 | $C_2H_5$ | $CH_2$=CH |
| 1.43 | $C_3H_7$-iso | $CH_2$=CH |
| 1.44 | $C_4H_9$-sek | $CH_2$=CH |
| 1.45 | $CH_3$ | $CH_2$=CH-$CH_2$ |
| 1.46 | $C_2H_5$ | $CH_2$=CH-$CH_2$ |
| 1.47 | $C_3H_7$-iso | $CH_2$=CH-$CH_2$ |
| 1.48 | $C_4H_9$-sek | $CH_2$=CH-$CH_2$ |
| 1.49 | $CH_3$ | CH≡C-$CH_2$ |
| 1.50 | $C_2H_5$ | CH≡C-$CH_2$ |
| 1.51 | $C_3H_7$-iso | CH≡C-$CH_2$ |
| 1.52 | $C_4H_9$-sek | CH≡C-$CH_2$ |
| 1.53 | $CH_3$ | $(CH_3)_2C$=CH |
| 1.54 | $C_2H_5$ | $(CH_3)_2C$=CH |
| 1.55 | $C_3H_7$-iso | $(CH_3)_2C$=CH |
| 1.56 | $C_4H_9$-sek | $(CH_3)_2C$=CH |
| 1.57 | $CH_3$ | $(Cl)_2C$=C(Cl) |
| 1.58 | $C_2H_5$ | $(Cl)_2C$=C(Cl) |
| 1.59 | $C_3H_7$-iso | $(Cl)_2C$=C(Cl) |
| 1.60 | $C_4H_9$-sek | $(Cl)_2C$=C(Cl) |
| 1.61 | $CH_3$ | $CF_3CCl_2$ |
| 1.62 | $C_2H_5$ | $CF_3CCl_2$ |
| 1.63 | $C_3H_7$-iso | $CF_3CCl_2$ |
| 1.64 | $C_4H_9$-sek | $CF_3CCl_2$ |
| 1.65 | $CH_3$ | Cyclopropyl |
| 1.66 | $C_2H_5$ | Cyclopropyl |
| 1.67 | $C_3H_7$-iso | Cyclopropyl |
| 1.68 | $C_4H_9$-sek | Cyclopropyl |
| 1.69 | $CH_3$ | 2,2-Dimethyl-cyclopropyl |
| 1.70 | $C_2H_5$ | 2,2-Dimethyl-cyclopropyl |
| 1.71 | $C_3H_7$-iso | 2,2-Dimethyl-cyclopropyl |
| 1.72 | $C_4H_9$-sek | 2,2-Dimethyl-cyclopropyl |

Tabelle (Fortsetzung)

| Verb. Nr. | $R_2$ | R |
|---|---|---|
| 1.73 | $CH_3$ | 2,2-Dimethyl-3-(2,2-dichlorvinyl)-cyclopropyl |
| 1.74 | $C_2H_5$ | 2,2-Dimethyl-3-(2,2-dichlorvinyl)-cyclopropyl |
| 1.75 | $C_3H_7$-iso | 2,2-Dimethyl-3-(2,2-dichlorvinyl)-cyclopropyl |
| 1.76 | $C_4H_9$-sek | 2,2-Dimethyl-3-(2,2-dichlorvinyl)-cyclopropyl |
| 1.77 | $CH_3$ | Cyclobutyl |
| 1.78 | $C_2H_5$ | Cyclobutyl |
| 1.79 | $C_3H_7$-iso | Cyclobutyl |
| 1.80 | $C_4H_9$-sek | Cyclobutyl |
| 1.81 | $CH_3$ | Cyclohexyl |
| 1.82 | $C_2H_5$ | Cyclohexyl |
| 1.83 | $C_3H_7$-iso | Cyclohexyl |
| 1.84 | $C_4H_9$-sek | Cyclohexyl |
| 1.85 | $CH_3$ | Phenyl |
| 1.86 | $C_2H_5$ | Phenyl |
| 1.87 | $C_3H_7$-iso | Phenyl |
| 1.88 | $C_4H_9$-sek | Phenyl |
| 1.89 | $CH_3$ | p-Chlorphenyl |
| 1.90 | $C_2H_5$ | p-Chlorphenyl |
| 1.91 | $C_3H_7$-iso | p-Chlorphenyl |
| 1.92 | $C_4H_9$-sek | p-Chlorphenyl |
| 1.93 | $CH_3$ | p-Tolyl |
| 1.94 | $C_2H_5$ | p-Tolyl |
| 1.95 | $C_3H_7$-iso | p-Tolyl |
| 1.96 | $C_4H_9$-sek | p-Tolyl |
| 1.97 | $CH_3$ | p-Nitrophenyl |
| 1.98 | $C_2H_5$ | p-Nitrophenyl |
| 1.99 | $C_3H_7$-iso | p-Nitrophenyl |
| 2.00 | $C_4H_9$-sek | p-Nitrophenyl |
| 2.1 | $CH_3$ | n-Hexyl |
| 2.2 | $C_2H_5$ | n-Hexyl |
| 2.3 | $C_3H_7$-iso | n-Hexyl |
| 2.4 | $C_4H_9$-sek | n-Hexyl |
| 2.5 | $CH_3$ | $ClCH_2C(CH_3)_2$ |
| 2.6 | $C_2H_5$ | $ClCH_2C(CH_3)_2$ |
| 2.7 | $C_3H_7$-iso | $ClCH_2C(CH_3)_2$ |
| 2.8 | $C_4H_9$-sek | $ClCH_2C(CH_3)_2$ |
| 2.9 | $CH_3$ | 1-Methylcyclopropyl |
| 2.10 | $C_2H_5$ | 1-Methylcyclopropyl |

Tabelle (Fortsetzung)

| Verb. Nr. | $R_2$ | R |
|---|---|---|
| 2.11 | $C_3H_7$-iso | 1-Methylcyclopropyl |
| 2.12 | $C_4H_9$-sek | 1-Methylcyclopropyl |
| 2.13 | $CH_3$ | Adamantyl |
| 2.14 | $C_2H_5$ | Adamantyl |
| 2.15 | $C_3H_7$-iso | Adamantyl |
| 2.16 | $C_4H_9$-sek | Adamantyl |
| 2.17 | $C_2H_5$ | p-Fluorphenoxymethyl |
| 2.18 | $C_2H_5$ | $ClC(CH_3)_2$ |
| 2.19 | $C_2H_5$ | $CH_3CCl_2$ |
| 2.20 | $C_2H_5$ | $CH_3CH_2C(CH_3)_2$ |
| 2.21 | $C_2H_5$ | $C(CH_3)_3CH_2$ |
| 2.22 | $C_2H_5$ | $C(CH_3)_3C(CH_3)_2$ |
| 2.23 | $C_2H_5$ | $ClCH_2$ |
| 2.24 | $C_2H_5$ | $CF_3CH_2$ |
| 2.25 | $C_2H_5$ | 1-Methylcyclobutyl |
| 2.26 | $C_2H_5$ | 1-Methylcyclopentyl |
| 2.27 | $C_2H_5$ | $FCH_2C(CH_3)_2$ |
| 2.28 | $C_2H_5$ | $CH_2=C(CH_3)$ |
| 2.29 | $C_2H_5$ | $ClCH_2CH_2$ |
| 2.30 | $C_2H_5$ | p-(tert.$C_4H_9$)phenyl |
| 2.31 | $C_2H_5$ | $CH_3CH_2CH_2$ |
| 2.32 | $C_2H_5$ | $CH_3CH_2$ |
| 2.33 | $C_2H_5$ | $CF_3C_6H_4$ |
| 2.34 | $C_2H_5$ | $C_6H_5(CH_3)CH$ |
| 2.35 | $C_2H_5$ | (S)-$C_6H_5(CH_3)CH$ |
| 2.36 | $C_2H_5$ | (R)-$C_6H(CH_3)CH$ |
| 2.37 | $C_2H_5$ | $C_6H_5(CH_3)_2C$ |
| 2.38 | $C_2H_5$ | $(CH_3CH_2CH_2)_2CH$ |
| 2.41 | $C_2H_5$ | $CH_3S(CH_3)_2C$ |
| 2.51 | $C_2H_5$ | o-(Trifluormethyl)phenyl |

Der Inhalt der Tabelle besitzt illustrativen Charakter und stellt keine Begrenzung dar. Weitere typische Vertreter sind Verbindungen der Formel I, in denen $R_2$ und R die in Tabelle I angegebenen Bedeutungen haben und $R_1$ für eine Alkyl-, Cycloalkyl- oder Acylgruppe steht.

Formulierungsbeispiele für den Wirkstoff der Formel I
(% = Gewichtsprozent)

F1. Spritzpulver

| | a) | b) | c) |
|---|---|---|---|
| Wirkstoff aus Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

F2. Emulsions-Konzentrat
Wirkstoff aus Tabelle 1 10 %
Octylphenolpolyethylenglykolether (4-5 Mol AeO) 3 %
Ca-Dodecylbenzolsulfonat 3 %
Ricinusölpolyglykolether (36 Mol AeO) 4 %
Cyclohexanon 30 %
Xylolgemisch 50 %

Aus diesem Konzentrat können durch Verdünnen mit Wasser Emulsionen jeder gewünschten Konzentration hergestellt werden.

F3. Stäubemittel

| | a) | b) |
|---|---|---|
| Wirkstoff aus Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

F4. Extruder Granulat
Wirkstoff aus Tabelle 1 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

F5. Tabletten, Pellets

I
Wirkstoff aus Tabelle 1 33,0 %
Methylcelluose 0,80 %
Kieselsäure hochdispers 0,80 %
Maisstärke 8,40 %

Methylcellulose in Wasser einrühren und quellen lassen; Kieselsäure in die Quellung einrühren und homogen suspendierten. Wirkstoff und Maisstärke mischen. In diese Mischung die wässrige Suspension einarbeiten und zu einem Teig kneten. Diese Masse durch ein Sieb (Maschenweite 12 M) granulierten und dann trocknen.

II
Milchzucker krist. 22,50 %
Maisstärke 17,00 %
mikrokrist. Cellulose 16,50 %

Magnesiumstearat 1,00 %
Alle 4 Hilfsstoffe gut mischen
Phasen I und II mischen und zu Tabletten oder Pellets verpressen.

Bei einer Verwendung von Verbindungen der Formel I oder entsprechender Mittel zur Bekämpfung von endoparasitären Nematoden, Cestoden und Trematoden bei Haus- und Nutztieren, wie Rindern, Schafen, Ziegen, Katzen und Hunden, können die Verbindungen oder Mittel den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichtung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Pellets, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die sie enthaltenden Mitteln den Tieren auch beispielsweise subcutan injiziert, intraruminal verabreicht oder mittels der Pour-on-Methode auf den Körper der Tiere appliziert werden. Ferner ist ein Verabreichung des Wirkstoffs an die Tiere auch durch Lecksteine (Salz) oder Molasse-Blöcke möglich.

Biologische Beispiele

B1. Wirkung gegen $L_1$-Laren von Lucilia sericata

1 ml einer wässrigen Suspension der zu prüfenden Aktivsubstanz werden, so mit 3 ml eines speziellen Larvenzuchtmediums bei ca. 50°C vermischt, dass ein Homogenisiert von wahlweise 250 ppm ode 100 ppm Wirkstoffgehalt entsteht. In jede Reagenzglas-Probe werden ca. 30 Lucilia-Larven ($L_1$) eingesetzt. Nach 4 Tagen wird die Mortalitätsräte bestimmt. Verbindungen der Formel I erzielen mit 250 ppm eine Wirkung von 100 %.

B2. Akarizide Wirkung gegen Boophilus microplus (Biarra-Stamm)

Auf einer PVC-Platte wird waagrecht ein Klebstreifen so befestigt, das darauf 10 mit Blut vollgesogene Zecken-Weibchen von Boophilus microplus (Biarra-Stamm) nebeinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine bestimmte Wirkstoffmenge gelöst ist, so das wahlweise 1, 0,5, 0,1 oder 0,01 µg pro Zecke appliziert werden. Kontrolltiere erhalten eine Injektion der entsprechenden Mischung ohne Wirkstoff. Nach der Behandlung werden die Tiere unter Normalbedingungen in einem Insektarium bei ca. 28°C und 80 % relativer Luftfeuchtigkeit gehalten, bis die Eiablage erfolgt und die Larvon aus den Eiern der Kontrolltieren geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zweckweibchen (= 90 %) Eier ablegen, die nicht schlupffähig sind.

Verbindungen der Formeln I erzielen eine $IR_{90}$ von 0,5 µg.

Die Verbindungen Nr. 1.10 (Tabelle 1) wirkt auch gegen die Nymphenstadien von Boophilus und anderen Zecken.

B3. Versuch an mit Nematoden (Haemonchus contortus und Trichostrongylus colubriformis) infizierten Schafen

Der Wirkstoff wird als Suspension formuliert mit einer Magensonde oder durch Injektion in den Pansen eines Schafes gegeben, das mit Haemonchus contortus und Trichostrongylus colubriformis künstlich infiziert worden ist. Pro Dosis werden 1 bis 3 Tiere verwendet. Jedes Schaf wird nur einmal mit einer einzigen Dosis behandelt, und zwar mit 0,5 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen der Formel I bei 0,5 mg/kg behandeltwerden, zeigen im Vergleich zu unbehandelten, aber infizierten Vergleichsgruppen keinen Nematodenbefall (= komplette Reduktion der Wurmeier im Kot).

B4. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Verbindungen der Formeln I bewirken in diesem Test bei einer Konzentration von 1,6 ppm bereits nach einem Tag eine vollständige Abtötung sämtlicher Larven.

B5. Milbizide Wirkung gegen Dermanyssus gallinae

2 bis 3 ml einer Testlösung (100, 10, 1 und 0,1 ppm Aktivsubstanz) werden in einen nach oben offenen Glasbehälter gegeben und ca. 200 Milben in unterschiedlichen Entwicklungsstadien in diesen Behälter eingesetzt. Der Glasbehälter wird mit einem Wattebausch verschlossen und 10 Minuten gleichmässig bis zur

vollständigen Benetzung der Milben geschüttelt. Danach wird der Behälter umgekehrt hingestellt, bis die überschüssige Testlösung von der Watte aufgesogen ist. Der Behälter wird erneut gekehrt und die behandelten Milben zur Evaluierung der Wirksamkeit der Testsubstanzen drei Tage unter Laborbedingungen beobachtet, wobei die Mortalität als Massstab für Wirksamkeit herangezogen wird.

Verbindungen aus den Herstellungsbeispielen zeigen bei 100 ppm eine Wirkung von 100 %.

**Patentansprüche**

1. Verbindungen der Formel I

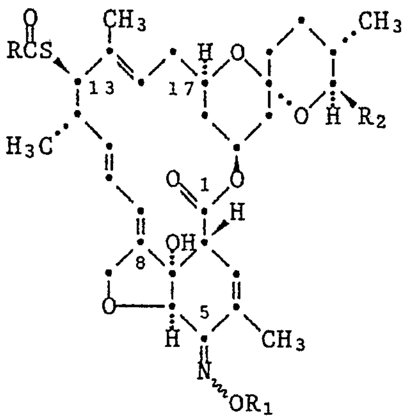

(I)

in welcher

$R_1$ Wasserstoff, eine Alkyl-, Cycloalkyl- oder Acylgruppe bedeutet, $R_2$ für Methyl, Ethyl, Isopropyl oder sek.Butyl oder für die Gruppe $-\underset{CH_3}{C}=CH-X$ steht, worin X Methyl, Ethyl oder Isopropyl bedeutet und R Wasserstoff, eine unsubstituierte oder substituierte gerad- oder verzweigtkettige $C_1$-$C_{18}$-Alkyl-Gruppe, eine unsubstituierte oder substituierte cycloaliphatische Gruppe mit 3 bis 10 Kohlenstoffatomen, eine unsubstituierte oder substituierte $C_2$-$C_6$-Alkenyl-Gruppe, eine unsubstituierte oder substituierte $C_2$-$C_6$-Alkinyl-Gruppe, eine unsubstituierte oder substituierte Phenyl-Gruppe oder eine unsubstituierte oder substituierte Benzylgruppe bedeutet.

2. Verbindungen der Formel Ia nach Anspruch 1

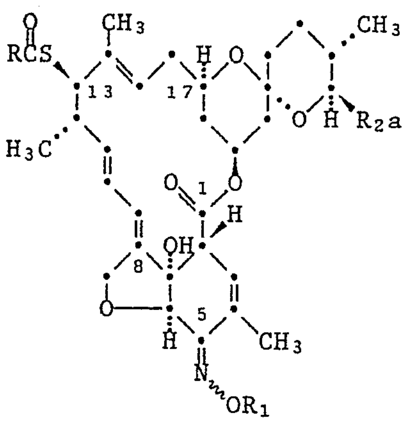

(Ia)

worin

$R_1$ Wasserstoff, eine Alkyl-, Cycloalkyl- oder Acylgruppe,

$R_{2}a$ Methyl, Ethyl, Isopropyl oder sek.Butyl und

R Wasserstoff, eine unsubstituierte oder substituierte gerad- oder verzweigtkettige $C_1$-$C_{18}$-Alkyl-Gruppe, eine unsubstituierte oder substituierte cycloaliphatische Gruppe mit 3 bis 10 Kohlenstoffatomen, eine unsubstituierte oder substituierte $C_2$-$C_2$-Alkenyl-Gruppe, eine unsubstituierte oder substituierte $C_2$-$C_6$-Alkinyl-Gruppe oder eine unsubstituierte oder substituierte Phenyl-Gruppe oder eine unsubstiterte oder substituierte Benzylgruppe bedeutet.

3. Verbindungen der Formel I nach Anspruch 1, in welchen die für R stehende Alkyl-, Cycloalkyl-, Alkenyl- und Alkinyl-Gruppe mit 1 bis 7 Halogenatomen oder 1 bis 6 $C_1$-$C_6$-Alkylthio- oder $C_1$-$C_8$-Alkoxygruppe und die Phenyl-Gruppe mit 1 bis 3 Substituenten, ausgewählt aus der Gruppe

bestehend aus Halogenatomen, $C_1$-$C_6$-Alkyl, $C_1$-$C_6$-Alkoxy, $C_1$-$C_6$-Alkylthio oder Nitro, substituiert ist, $R_2$ für Methyl, Ethyl, Isopropyl oder sec-Butyl steht und $R_1$ die unter Formel I in Anspruch 1 angegebenen Bedeutungen besitzt.

4. Verbindungen der Formel I nach Anspruch 1, worin $R_1$ Wasserstoff darstellt, $R_2$ für Methyl, Ethyl, Isopropyl oder sek.Butyl steht und R Wasserstoff, unsubstituiertes oder durch 1 bis 4 Halogenatome, $C_1$-$C_4$-Alkylthio oder $C_1$-$C_4$-Alkoxy substituiertes $C_1$-$C_6$-Alkyl, $C_2$-$C_4$-Alkenyl, $C_2$-$C_4$-Alkinyl oder $C_3$-$C_6$-Cycloalkyl; oder unsubstituiertes oder ein- bis dreifach durch Halogen, $C_1$-$C_4$-Alkyl, $C_1$-$C_4$-Alkoxy, $C_1$-$C_4$-Alkylthio oder Nitro substituiertes Phenyl oder Benzyl bedeutet.

5. Verbindungen der Formel I nach Anspruch 1, worin $R_1$ Wasserstoff darstellt, $R_2$ für Methyl, Ethyl, Isopropyl oder sek.Butyl steht und R Wasserstoff, unsubstituiertes oder durch 1 bis 4 Chlor- oder Fluroatome, Methylthio oder Methoxy substituiertes $C_1$-$C_4$-Alkyl, $C_2$-$C_3$-Alkenyl, $C_2$-$C_3$-Alkinyl oder $C_3$-$C_6$-Cycloalkyl; oder unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl oder Benzyl bedeutet.

6. Verbindungen der Formel I nach Anspruch 1, worin $R_1$ Wasserstoff darstellt, $R_2$ für Ethyl oder Methyl steht und R Wasserstoff, $C_1$-$C_8$-Alkyl, welches unsubstituiert oder durch $C_1$-$C_4$-Alkoxy, $C_1$-$C_4$-Alkylthio oder ein- bis dreifach halogeniertes Phenoxy monosubstituiert oder durch 1 bis 5 Halogenatome substituierte ist, eine substituierte oder durch Substituenten aus der Gruppe bestehend aus $C_1$-$C_4$-Alkyl und halogeniertem $C_2$-$C_4$-Alkenyl ein- oder mehrfach substituierte, mono-bis tetracyclische, aliphatische Gruppe mit insgesamt 3 bis 10 Kohlenstoffatomen im Ring oder Ringsystem, ein- bis dreifach halogeniertes $C_2$-$C_4$-Alkenyl, $C_3$-$C_4$-Alkinyl oder ein- bis dreifach durch Substituenten aus der Gruppe bestehend aus Halogen, $C_1$-$C_4$-Alkyl und Nitro substituiertes Phenyl oder Benzyl bedeutet.

7. Verbindungen der Formel I nach Anspruch 1, worin $R_1$ Wasserstoff darstellt, $R_2$ für Ethyl oder Methyl steht und R Wasserstoff, $C_1$-$C_8$-Alkyl, welches unsubstituiert oder durch Substituenten der Gruppe bestehend aus Chlor und Fluor ein- bis dreifach substituiert ist, Fluorphenoxymethyl, unsubstituiertes oder durch eine Methylgruppe substituiertes $C_3$-$C_4$-Cycloalkyl, Adamantyl, Trichlorvinyl, Phenyl oder Monochlorphenyl bedeutet.

8. Verbindungen der Formel Ia nach Anspruch 2, worin $R_1$ einen Alkyl-oder Cycloalkyl-Rest darstellen.

9. Verbindungen der Formel Ia nach Anspruch 2, worin $R_1$ einen Rest aus der Gruppe Methyl, Ethyl, Propyl, Cyclopropyl, Cyclobutyl, Cyclopentyl, Cyclohexyl bedeutet.

10. Verbindungen der Formel I nach Anspruch 1, worin $R_1$ den Rest $R_3$-C(O)- darstellt, wobei $R_3$ $C_1$-$C_{10}$-Alkyl, $C_1$-$C_{10}$-Haloalkyl oder einen unsubstituierten oder durch Substituenten der Gruppe bestehend aus Halogen, $C_1$-$C_3$-Alkyl, $C_1$-$C_3$-Haloalkyl, $C_1$-$C_3$-Alkoxy, $C_1$-$C_3$-Haloalkoxy, Cyano und Nitro substituierten Rest aus der Gruppe Phenyl und Benzyl bedeutet, $R_2$ für Methyl, Ethyl, Isopropyl oder sec-Butyl steht und R die in Anspruch 1 angegebene Bedeutung hat.

11. Verbindung nach Anspruch 1, ausgewählt aus der Gruppe:
13β-Formylthio-5-oximino-milbemycin D
13β-Acetylthio-5-oximino-milbemycin D
13β-Pivaloylthio-5-oximino-milbemycin D
13β-Formylthio-5-oximino-milbemycin $A_3$
13β-Acetylthio-5-oximino-milbemcyin $A_3$
13β-Pivaloylthio-5-oximino-milbemycin $A_3$
13β-Formlythio-5-oximino-milbemycin $A_4$
13β-Acetylthio-5-oximino-milbemycin $A_4$
13β-Pivaloylthio-5-oximino-milbemycin $A_4$.
13β-(2′-Methoxy-2′-methylpropionylthio)-5-oximino-milbemycin D
13β-(2′-Methoxy-2′-methylpropionylthio)-5-oximino-milbemycin $A_4$
13β-Trichloracetylthio-5-oximino-milbemycin $A_4$
13β-(4′-Chlor-butanoylthio)-5-oximino-milbemycin $A_4$
13β-Trichloracryloylthio-5-oximino-milbemycin $A_4$
13β-Cyclopropancarbonylthio-5-oximino-milbemycin $A_4$
13β-Cyclobutancarbonylthio-5-oximino-milbemycin $A_4$
13β-Heptanoylthio-5-oximino-milbemycin $A_4$
13β-(3′-Chlor-2′,2′-dimethyl-propionyltho)-5-oximino-milbemycin $A_4$
13β-(3′-Chlor-2′,2′-dimethyl-propionylthio)-5-oximino-milbemycin $A_3$
13β-(1′-Methyl-cyclopropancarbonylthio)-5-oximino-milbemycin $A_4$
13β-(1′-Methyl-cyclopropancarbonylthio)-5-oximino-milbemycin $A_3$
13β-(1-Adamantancarbonylthio)-5-oximino-milbemycin $A_4$
13β-(p-Fluorphenoxyacetylthio)-5-oximino-milbemycin $A_4$
13β-(2′-Chlor-2′-methyl-propionylthio)-5-oximino-milbemycin $A_4$
13β-(2′,2′-Dichlorpropionylthio)-5-oximino-milbemycin $A_4$
13β-(2′,2′-Dimethylbutanoylthio)-5-oximino-milbemycin $A_4$
13β-(3′,3′-Dimethylbutanoylthio)-5-oximino-milbemycin $A_4$
13β-(2′,2′,3′,3′-Tetramethylbutanoylthio)-5-oximino-milbemycin $A_4$
13β-(p-Chlorbenzoylthio)-5-oximino-milbemycin $A_4$
13β-(3′,3′,3′-Trifluorpropionylthio)-5-oximino-milbemycin $A_4$

13β-Chloracetylthio-5-oximino-milbemycin $A_4$
13β-(2′-Chlor-3′,3′,3′-trifluorpropionylthio)-5-oximino-milbemycin $A_4$
13β-(3′,3′,3′-Trifluorpropionylthio)-5-oximino-milbemycin $A_4$
13β-(4′-Heptylcarbonylthio)-5-oximino-milbemycin $A_4$
13β-(4′-Heptylcarbonylthio)-5-oximino-milbemycin $A_3$
13β-(2′-Trifluormethylbenzoylthio)-5-oximino-milbemycin $A_4$
13β-(2′-Trifluormethylbenzoylthio)-5-oximino-milbemycin $A_3$
13β-((R/S)-2′-Phenylpropionylthio)-5-oximino-milbemycin $A_4$
13β-((R/S)-2′-Phenylpropionylthio)-5-oximino-milbemycin $A_3$
13β-(2,2′-Dimethylbutyrylthio)-5-oximino-milbemycin $A_4$
13β-(2,2′-Dimethylbutyrylthio)-5-oximino-milbemycin $A_3$
13β-(3′-Fluor-2′,2′-dimethylpropionylthio)-5-oximino-milbemycin $A_4$
13β-(3′-Fluor-2′,2′-dimethylpropionylthio)-5-oximino-milbemycin $A_3$
13β-(Methoxiacetylthio)-5-oximino-milbemycin $A_4$
13β-(Methoxiacetylthio)-5-oximino-milbemycin $A_3$
13β-(2′3′-(Difluormethylendioxibenzoylthio-5-oximino-milbemycin $A_4$
13β-(2′3′-(Difluormethylendioxibenzylthio-5-oximino-milbemycin $A_3$.

12. Verfahren zur Herstellung von Verbindungen der Formel I

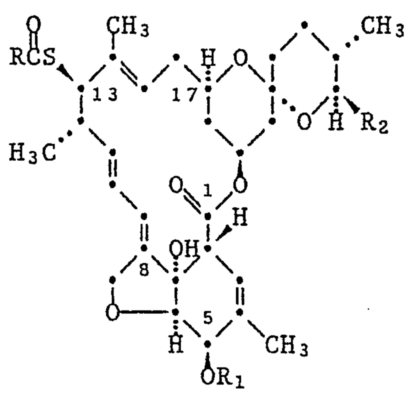

(I)

in welcher
$R_1$ Wasserstoff, eine Alkyl-, Cycloalkyl- oder Acylgruppe bedeutet
$R_2$ für Methyl, Ethyl, Isopropyl oder sek.Butyl oder für die Gruppe $-\underset{CH_3}{C}=CH-X$ steht, worin X Methyl, Ethyl odr Isopropyl bedeutet, und R Wasserstoff, eine unsubstituierte oder substituierte gerad- oder verzweigtkettige $C_1$-$C_{18}$-Alkyl-Gruppe, eine unsubstituierte oder substituierte cycloaliphatische Gruppe mit 3 bis 10 Kohlenstoffatomen, eine unsubstituierte oder substituierte $C_2$-$C_6$-Alkenyl-Gruppe, eine unsubstituierte oder substituierte $C_2$-$C_6$-Alkinyl-Gruppe, eine unsubstituierte oder substituierte Phenyl-Gruppe oder eine unsubstituierte oder substituierte Benzylgruppe bedeuet, dadurch gekennzeichnet, dass man Verbindung der Formel II

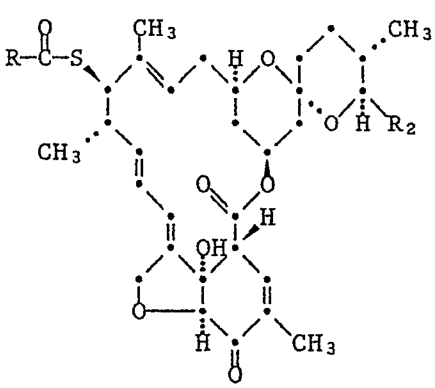

(II)

worin R und $R_2$ die unter Formel I angegebene Bedeutungen haben, mit einem geeigneten Oximinierungsreagenz umsetzt.

13. Verfahren nach Anspruch 12 zur Herstellung von Verbindungen der Formel Ia

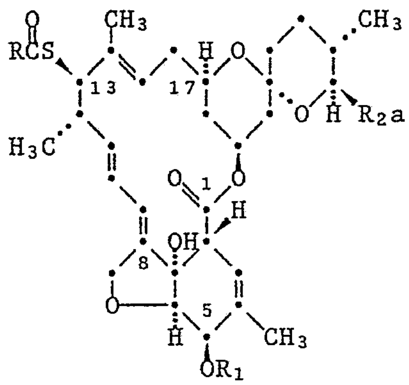

(Ia)

in welcher
$R_1$ Wasserstoff, eine Alkyl-, Cycloalkyl- oder Acylgruppe bedeutet
$R_{2}a$ für Methyl, Ethyl, Isopropyl oder sek.Butyl steht, und
R Wasserstoff, eine unsubstituierte oder substituierte gerad- oder verzweigtkettige $C_1$-$C_{18}$-Alkyl-Gruppe, eine unsubstituierte oder substituierte cycloaliphatische Gruppe mit 3 bis 10 Kohlenstoffatomen, eine unsubstituierte oder substituierte $C_2$-$C_6$-Alkenyl-Gruppe, eine unsubstituierte oder substituierte $C_2$-$C_6$-AlkinylGruppe, eine unsubstituierte oder substituierte Phenyl-Gruppe oder eine unsubstituierte oder substituierte Benzylgruppe bedeuet, dadurch gekennzeichnet, dass man Verbindung der Formel II

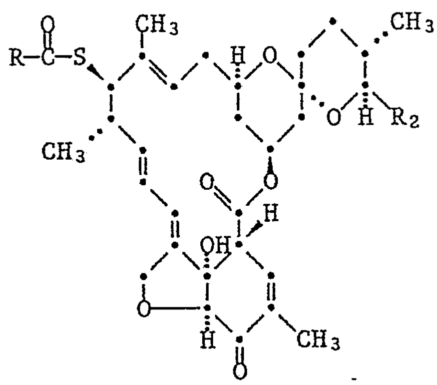

(II)

worin $R_2$ für $R_{2}a$ steht und R und $R_{2}a$ die unter Formel Ia angegebene Bedeutungen haben, mit einem geeigneten Oximierungsreagenz umsetzt.

14. Verfahren nach einem der Ansprüche 12 oder 13, dadurch gekennzeichnet, dass man als Oximierungsreagenz Hydroxylamin oder eines seiner Derivate der Formel $NH_2OR_1$ einsetzt, worin $R_1$ die unter Formel I angegebenen Bedeutungen hat oder, dass man ein davon abgeleitetes Mineralsäuresalz einsetzt.

15. Verfahren nach einem der Ansprüche 12 bis 14, dadurch gekennzeichnet, dass man die Umsetzung bei Temperaturen von 0° bis 80° C durchführt.

16. Verfahren nach Anspruch 15, dadurch gekennzeichnet, dass man bis 20° bis 40° arbeitet.

17. Verfahren zur Herstellung von Verbindungen der Formel I und Ia, worin $R_1$ für eine Acylgruppe der Formel $R_3$-C(O)- steht, worin $R_3$ für $C_1$-$C_{10}$-Alkyl, $C_1$-$C_{10}$-Haloalkyl oder einen unsubstituierten oder durch Substituenten der Gruppe bestehend aus Halogen, $C_1$-$C_3$-Alkyl, $C_1$-$C_3$-Haloalkyl, $C_1$-$C_3$-Alkoxy, $C_1$-$C_3$-Haloalkoxy, Cyano und Nitro substituierten Rest aus der Gruppe Phenyl und Benzyl steht und vorzugsweise $C_1$-$C_6$-Alkyl, $C_1$-$C_6$-Haloalkyl oder unsubstituiertes oder durch Halogen, $C_1$-$C_3$-Alkyl, $CF_3$ oder Nitro substituiertes Phenyl bedeutet, dadurch gekennzeichnet, dass man eine Verbindung der Formel I, worin $R_1$ für Wasserstoff steht, mit einem Säurehalogenid der Formel III

$R_3-\overset{O}{\overset{\|}{C}}-Hal$ (III)

umsetzt, worin $R_3$ die obigen Bedeutungen hat und Hal für Halogen steht.

18. Verfahren zur Bekämpfung von Schädlingen, dadurch gekennzeichnet, dass man eine pestizid-wirksame Menge mindestens einer Verbindung der Formel I oder Ia gemäss einem der Ansprüche 1 bis 11 auf oder in die Wirtstiere, auf Pflanzen oder den Lebensraum der Schädlinge appliziert.

19. Verfahren nach Anspruch 18, dadurch gekennzeichnet, dass die zu bekämpfenden Schädlinge Tiere befallende Endo- oder Ektoparasiten sind.

20. Verbindung der Formel II

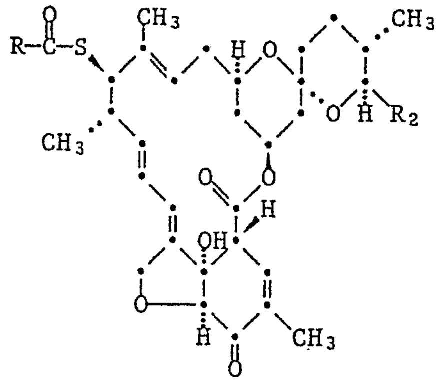

(II)

worin
$R_2$ für Methyl, Ethyl, Isopropyl oder sek.Butyl oder für die Gruppe $-\underset{CH_3}{C}=CH-X$ steht, worin X Methyl, Ethyl oder Isopropyl bedeutet und R Wasserstoff, eine unsubstituierte oder substituierte gerad- oder verzweigtkettige $C_1$-$C_{18}$-Alkyl-Gruppe, eine unsubstituierte oder substituierte cycloaliphatisch Gruppe mit 3 bis 10 Kohlenstoffatomen, eine unsubstituierte oder substituierte $C_2$-$C_6$-Alkenyl-Gruppe, eine unsubstituierte oder substituierte $C_2$-$C_6$-Alkinyl-Gruppe, eine unsubstituierte oder substituierte Phenyl-Gruppe oder eine unsubstituierte oder substituierte Benzylgruppe bedeutet.

21. Verbindungen der Formel II nach Anspruch 20

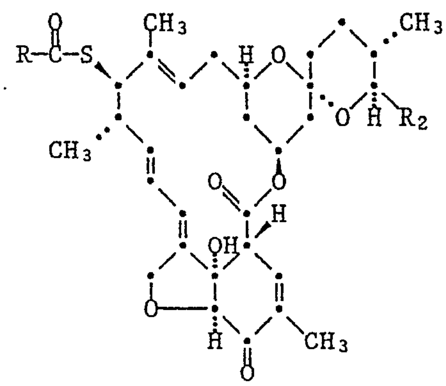

(II)

worin
$R_2$ für Methyl, Ethyl, Isopropyl oder sek.Butyl steht und R Wasserstoff, eine unsubstituierte oder substituierte gerad- oder verzweigtkettige $C_1$-$C_8$-Alkyl-Gruppe, eine unsubstituierte oder substituierte cycloaliphatische Gruppe mit 3 bis 10 Kohlenstoffatomen, eine unsubstituierte oder substituierte $C_2$-$C_6$-Alkenyl-Gruppe, eine unsubstituierte oder substituierte $C_2$-$C_6$-Alkinyl-Gruppe, eine unsubstituierte oder substituierte Phenyl-Gruppe oder eine unsubstituierte oder substituierte Benzylgruppe bedeutet.

22. Verfahren zur Herstellung einer Veribndung der Formel II

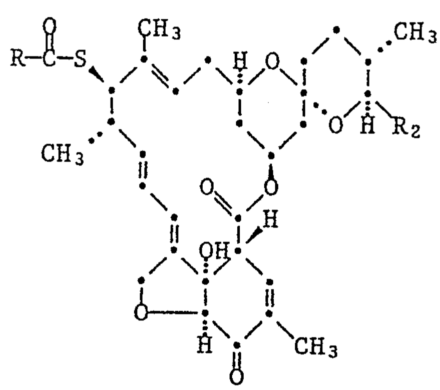

(II)

worin

$R_2$ für Methyl, Ethyl, Isopropyl oder sek.Butyl oder für die Gruppe 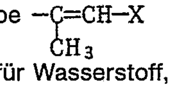 steht, worin X Methyl, Ethyl oder Isopropyl bedeutet und R für Wasserstoff, eine unsubstituierte oder substituierte gerad-oder verzweigtkettige $C_1$-$C_{18}$-Alkyl-Gruppe, eine unsubstituierte oder substituierte cycloaliphatische Gruppe mit 3 bis 10 Kohlenstoffatomen, eine unsubstituierte oder substituierte $C_2$-$C_6$-Alkenyl-Gruppe, eine unsubstituierte oder substituierte $C_2$-$C_6$-Alkinyl-Gruppe, eine unsubstituierte oder substituierte Phenyl-Gruppe oder eine unsubstituierte oder substituierte Benzylgruppe steht, dadurch gekennzeichnet, dass man eine Verbindung der Formel IV

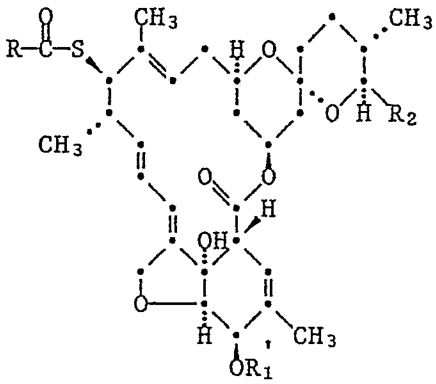

(IV)

worin

R Wasserstoff bedeutet und R und $R_2$ die unter Formel II angegebenen Bedeutungen haben, in einem reaktionsinerten Lösungsmittel bei Temperaturen von -80° bis +60° C oxidiert.

23. Verfahren zur Herstellung einer Verbindung der Formel II nach Anspruch 22,

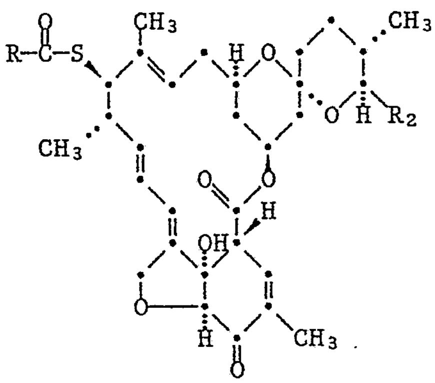

(II)

worin

$R_2$ für Methyl, Ethyl, Isopropyl oder sek.Butyl steht und

R für Wasserstoff, eine unsubstituierte oder substituierte gerad-oder verzweigtkettige $C_1$-$C_{18}$-Alkyl-Gruppe, eine unsubstituierte oder substituierte cycloaliphatische Gruppe mit 3 bis 10 Kohlenstoffatomen, eine unsubstituierte oder substituierte $C_2$-$C_6$-Alkenyl-Gruppe, eine unsubstituierte oder substituierte $C_2$-$C_6$-Alkinyl-Gruppe, eine unsubstituierte oder substituierte Phenyl-Gruppe oder eine unsubstituierte oder substituierte Benzylgruppe steht, dadurch gekennzeichnet, dass man eine Verbindung der Formel IV

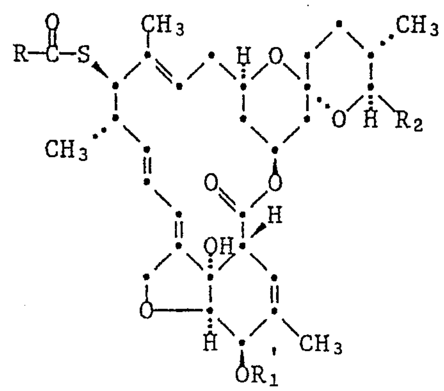

(IV)

worin
$R_1$ Wasserstoff bedeutet, $R_2$ für Methyl, Ethyl, Isopropyl oder sek.Butyl steht und R die unter Formel II angegebenen Bedeutungen haben, in einem reaktionsinerten Lösungsmittel bei Temperaturen von -80° bis +60° C oxidiert.

24. Verwendung einer Verbindung der Formel II gemäss Anspruch 20 zur Herstellung einer Verbindung der Formel I gemäss Anspruch 1.

25. Verwendung einer Verbindung der Formel II gemäss Anspruch 21 zur Herstellung einer Verbindung der Formel Ia gemäss Anspruch 2.

26. Schädlingsbekämpfungsmittel zur Bekämpfung von Ekto- und Endoparasiten und Insekten, dadurch gekennzeichnet, dass neben den üblichen Trägermitteln, Verteilungsmitteln oder Träger- und Verteilungsmitteln als Wirkstoff mindestens eine Verbindung der Formel I

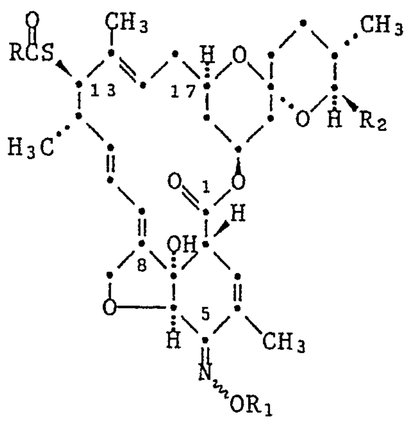

(I)

in welcher
$R_1$ Wasserstoff, eine Alkyl-, Cycloalkyl- oder Acylgruppe bedeutet,
$R_2$ für Methyl, Ethyl, Isopropyl oder sek.Butyl oder für die Gruppe $-\underset{CH_3}{\underset{|}{C}}=CH-X$ steht,
worin X Methyl, Ethyl oder Isopropyl bedeutet und R Wasserstoff, eine unsubstituierte oder substituierte gerad- oder verzweigtkettige $C_1$-$C_{18}$-Alkyl-Gruppe, eine unsubstituierte oder substituierte cycloaliphatische Gruppe mit 3 bis 10 Kohlenstoffatomen, eine unsubstituierte oder substituierte $C_2$-$C_6$-Alkenyl-Gruppe, eine unsubstituierte oder substituierte $C_2$-$C_6$-Alkinyl-Gruppe, eine unsubstituierte oder substituierte Phenyl-Gruppe oder eine unsubstituierte oder substituierte Benzylgruppe bedeutet, enthält.

27. Mittel nach Anspruch 26, dadurch gekennzeichnet, dass es neben den üblichen Trägermitteln, Verteilungsmitteln oder Träger- und Vertilungsmitteln als Wirkstoff mindestens eine Verbindung der Formel Ia

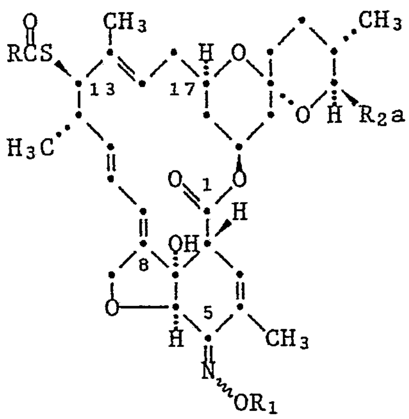

(Ia)

worin

$R_1$ Wasserstoff, eine Alkyl-, Cycloalkyl- oder Acylgruppe,

$R_{2}a$ Methyl, Ethyl, Isopropyl oder sek.Butyl und

R Wasserstoff, eine unsubstituierte oder substituierte gerad- oder verzweigtkettige $C_1$-$C_{18}$-Alkyl-Gruppe, eine unsubstituierte oder substituierte cycloaliphatische Gruppe mit 3 bis 10 Kohlenstoffatomen, eine unsubstituierte oder substituierte $C_2$-$C_6$-Alkenyl-Gruppe, eine unsubstituierte oder substituierte $C_2$-$C_6$-Alkinyl-Gruppe oder eine unsubstituierte oder substituierte Phenyl-Gruppe oder eine unsubstituierte oder substituierte Benzylgruppe bedeutet, enthält.

28. Mittel nach Anspruch 26, dadurch gekennzeichnet, dass es als Verbindung der Formel I mindestens eine Verbindung gemäss einem der Ansprüche 3 bis 11 enthält.

29. Eine Verbindung der Formel I gemäss einem der Ansprüche 1 und 3 bis 11, zur Bekämpfung von Ekto- und Endoparasiten an Pflanzen und Tieren.

30. Eine Verbindung der Formel Ia gemäss Anspruch 2 zur Bekämpfung von Ekto- und Endoparasiten an Pflanzen und Tieren.

31. Eine Verbindung der Formel I gemäss Anspruch 29 zur Bekämpfung von Endoparasiten im Warmblüter.

32. Eine Verbindung der Formel Ia gemäss Anspruch 30 zur Bekämpfung von Endoparasiten im Warmblüter.

33. Eine Verbindung der Formel I gemäss Anspruch 29 zur Bekämpfung von Ektoparasiten an Tieren oder von Pflanzenparasiten, die man in Aufwandmengen von 10 g bei 1000 g per Hektar auf geschlossene Kulturanbauflächen, in Pferchen, Stallungen oder sonstigen Räumen appliziert.

34. Eine Verbindung der Formel Ia gemäss Anspruch 30 zur Bekämpfung von Ektoparasiten an Tieren oder von Pflanzenparasiten, die man in Aufwandmengen von 10 g bei 1000 g per Hektar auf geschlossene Kulturanbauflächen, in Pferchen, Stallungen oder sonstigen Räumen appliziert.

Europäisches Patentamt

# EUROPÄISCHER RECHERCHENBERICHT

Nummer der Anmeldung

EP 88 81 0088

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 184 173 (CIBA-GEIGY) * Ansprüche 1, 20-23; Seiten 37, 38, Tabelle 1, Verbindungen Nr. 1.25-1.28, 1.33-1.36 * | 1,18-21,26 | C 07 D 493/22<br>A 01 N 43/90<br>A 61 K 31/35 //<br>(C 07 D 493/22<br>C 07 D 313:00<br>C 07 D 311:00<br>C 07 D 311:00<br>C 07 D 307:00 ) |
| A | EP-A-0 110 667 (SANKYO CO. LTD.) * Ansprüche 1, 11-13 * | 1,12,13,18,26 | |
| A | EP-A-0 203 832 (SANKYO CO. LTD.) * Ansprüche 1, 3, 4, 12 * | 1,18,26 | |
| A | CHEMICAL ABSTRACTS, Band 104, Nr. 13, 31. März 1986, Seiten 724, 725, Zusammenfassungsnr. 109603r, Columbus, Ohio, US; & JP - A - 60 142 991 | 1,18,26 | |
| A | EP-A-0 186 043 (CIBA-GEIGY AG) * Ansprüche 1, 17 * | 20,21,26 | |
| | | | **RECHERCHIERTE SACHGEBIETE (Int. Cl.4)** |
| | | | C 07 D 493/00<br>C 07 H 19/00 |

Der vorliegende Recherchenbericht wurde für alle Patentansprüche erstellt

| Recherchenort | Abschlußdatum der Recherche | Prüfer |
|---|---|---|
| BERLIN | 10-05-1988 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)